# EUROPEAN PATENT SPECIFICATION

(11) **EP 0 882 696 B1**
(45) Date of publication and mention of the grant of the patent: **24.01.2001**
(21) Application number: 96938452.8
(22) Date of filing: 13.11.1996
(51) Int. Cl.: C07C 43/188, C07C 43/192, C07C 43/215, C07C 43/225, C07C 255/46, C07C 255/54, C09K 19/20, C09K 19/30, C09K 19/42, G02F 1/13

(54) **ALKENYL COMPOUNDS BEARING OXYGENIC LINKAGES, LIQUID-CRYSTAL COMPOSITION, AND LIQUID-CRYSTAL DISPLAY ELEMENT**
ALKENYLVERBINDUNGEN MIT SAUERSTOFFBINDUNGEN, FLUSSIGKRISTALLINE ZUSAMMENSETZUNGEN UND FLUSSIGKRISTALLINES ANZEIGEELEMENT
COMPOSES ALCENYLE COMPORTANT DES LIAISONS OXYGENE, COMPOSITION DE CRISTAUX LIQUIDES ET ELEMENT D'AFFICHAGE A CRISTAUX LIQUIDES

(30) Priority: 05.12.1995 JP 34495695
(43) Date of publication of application: 09.12.1998
(73) Proprietor: CHISSO CORPORATION, Osaka-shi Osaka 530-6591 (JP)
(72) Inventor: KONDO, Tomoyuki, Ichihara-shi, Chiba-ken 290 (JP); MATSUI, Shuichi, Ichihara-shi, Chiba-ken 290 (JP); MIYAZAWA, Kazutoshi, Ichihara-shi, Chiba-ken 290-01 (JP); HACHIYA, Norihisa, Ichihara-shi, Chiba-ken 299-01 (JP); NAKAGAWA, Etsuo, Ichihara-shi, Chiba-ken 290 (JP)
(74) Representative: Walter, Wolf-Jürgen
(86) International application number: JP9603323
(87) International publication number: WO9720791

(56) References cited:
- EP-A- 0 315 014
- EP-A- 0 331 933
- EP-A- 0 344 557
- EP-A- 0 377 516
- JP-A- 1 151 531
- JP-A- 2 180 840
- JP-A- 8 067 641
- JP-A- 8 157 409
- JP-A- 61 227 547
- US-A- 5 271 864
- PATENT ABSTRACTS OF JAPAN vol. 11, no. 69 (C-407), 3 March 1987 & JP 61 227547 A (ASAHI GLASS CO. LTD.), 9 October 1986
- STEPHEN M. KELLY: "50. Ferroelectric liquid crystals. Part 3" HELVETICA CHIMICA ACTA., vol. 71, no. 2, 1988, pages 461-466, XP002093774 BASEL CH

## Description

The present invention relates to liquid crystalline compounds and liquid crystal compositions, more particularly, it relates to new alkenyl compounds having bond groups containing oxygen atoms, liquid crystal compositions containing the liquid crystalline compounds, and liquid crystal display devices constituted by using the liquid crystal compositions.

### Background Art

Liquid crystal devices are broadly used in clocks, watches and electronic calculators, various measuring apparatus, panels for automobiles, word processors, electronic notebooks, printers, computers, televisions and the like.

To these display devices, liquid crystalline compounds having an optical anisotropy (Δn) or a dielectric anisotropy (Δε) are applied. As such display modes, for example, dynamic scattering (DS), guest-host (GH), twisted nematic (TN), super twisted nematic (STN), thin film transistor (TFT), and ferroelectric liquid crystal (FLC) modes are known. As driving systems, for example, static, time sharing, active matrix and dual frequency driving systems are known. In these systems, particularly, the STN mode is in general used, because the display efficiency is very high and the production cost is low.

The liquid crystal compounds used in the STN mode need the following special properties:
1) a steep threshold voltage (Vₜₕ);
2) a wide distributed driving temperature (having good miscibility to the other liquid crystalline compounds);
3) short response time; and
4) low driving voltage.

In these properties, 1) is attained by a higher value of elastic constant ratio (particularly, K₃₃/K₁₁) (Proc. of the Japan Display, 388 (1986)). By using the liquid crystal compositions containing such compounds having the above properties, it is possible to obtain liquid crystal devices having high contrast ratio and good display quality, so that the special property is most important in the above properties.

2) is a property necessary for using the liquid crystal devices (like the liquid crystal compositions used for the devices) in the broad range of temperatures. It is required that the liquid crystal compositions have a nematic phase in the broad range of temperatures, particularly at a low temperature, and crystallized and smectic phases do not develop. However, since the liquid crystal compositions are obtained by mixing a few to twenties kinds of liquid crystalline compounds according to necessary characteristics of each display device, to satisfy the above demand, it is very important that the liquid crystalline compound mixed with the other liquid crystal compounds shows good compatibility particularly at a low temperature.

3) means that the response time is short to the electric field of liquid crystal molecules which orients in a liquid crystal panel. It is known that since the property is controlled by the viscosity of the liquid crystal composition, the viscosity may be merely lowered, Phys. Lett., 39A. 69 (1972). The liquid crystal compositions showing such rapid response time can be prepared by using much liquid crystalline compounds having very low viscosity. In this case, retardation (1st minimum conditions: Appl. Phys. Lett. 38 (7), 497) is considerable from the display quality. Namely, to hold the constant product of cell thickness and Δn (composition), it needs to select the liquid crystalline compounds so as to maintain the Δn in the proper value.

4) means that the Vₜₕ of compositions, which is very important to design liquid crystal devices, is minimized. The Vₜₕ is a function of Δε ( Vₜₕ = π(K/ε ₀Δε)^{1/2} , Mol. Cryst. Liq. Cryst., 12, 57 (1970)). Owing to the reason, it needs liquid crystalline compounds having Δε in proper value according to the purpose, and liquid crystal compositions containing such liquid crystalline compounds.

In addition to the above special properties, the following special properties are required.

The first is stability. Since liquid crystal devices are used in severe conditions such as high temperatures or outdoors, the liquid crystalline compounds for liquid crystal compositions should have sufficiently high chemical stability.

The second is temperature dependence in each item of physical special properties.

Namely, practical liquid crystal devices need to maintain the display quality under various environmental conditions, particularly, within a very broad range of temperatures, for example -20 - 40°C. For this reason, it needs to use liquid crystal compositions not having or having very low temperature dependence in each value of physical properties, or liquid crystalline compounds to be contained in such compositions.

To response such demand, hitherto, various liquid crystalline compounds for STN have been studied. From such study, an alkenyl compound has been found as a compound having relatively high K₃₃/K₁₁, and it is used for liquid crystal compositions and liquid crystal devices. As an example, the two rings system p type liquid crystal represented by formula (10) (Δε is positive) is disclosed in Mol. Cryst. Liq. Cryst., 122, 241 (1985), and the two rings system n type liquid crystal represented by formula (II) (Δε is negative) is disclosed by Japanese Patent Application Unexamined Laid-open No. 1-151531.

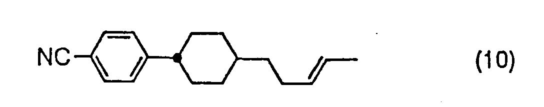

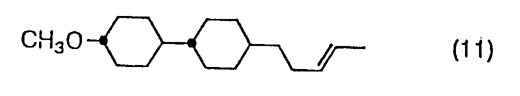

In addition, as a compound showing a nematic phase at the high temperature region, a three rings system compound represented by formula (12) is disclosed by Japanese Patent Application Unexamined Laid-open No. 61-83136.

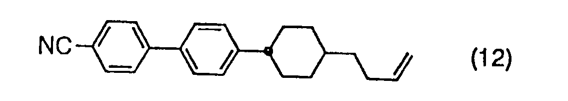

However, these compounds represented by formulas (10)-(12) have insufficiently high K₃₃/K₁₁, and these are not always satisfied with the viscosity, the compatibility in the other liquid crystalline compounds and temperature characteristics in each value of physical properties.

Objects of the present invention are to dissolve the problems of the above conventional techniques, and to provide new liquid crystalline compounds having characteristics, such as a steep Vₜₕ, good miscibility to the other liquid crystalline compound, low viscosity, rapid responsibility, and appropriate magnitude of Δn and Δε, and to provide liquid crystal compositions containing these compounds.

The inventors of the present invention have achieved the invention by finding that liquid crystalline compounds having the above properties are obtained by molecules having conventionally known alkenyl groups as an example of end groups and further groups selected from the group consisting of methylene oxy groups, oxy methylene groups, butylene oxy groups and oxy butylene groups as central bond groups.

### Disclosure of Invention

For achieving the above objects, the present invention is characterized by the following constitution:
(1) An alkenyl compound represented by general formula (1): 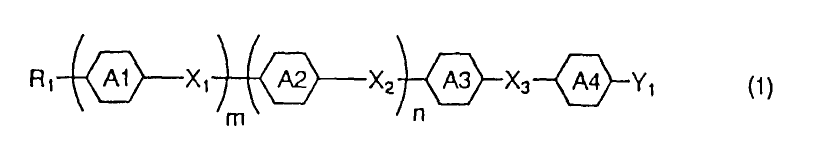 wherein X₁, X₂, and X₃ each independently represents -CH₂O-, -OCH₂-, -(CH₂)₃O-, -O(CH₂)₃-, a single bond, -CF₂O-, -OCF₂-, -CH=CH-, -C≡C-, .CH₂CH₂-, -(CH₂)₄-, -CH=CH-CH₂CH₂- or -CH₂CH₂-CH=CH-, and at least one indicates - -CH₂O-, -OCH₂-, -(CH₂)₃O- or -O(CH₂)₃-; R₁ represents an alkenyl group of 2-20 carbon atoms, Y₁ represents a fluorine atom, a chlorine atom, a bromine atom, an iodine atom, a cyano group or an alkyl group of 1-20 carbon atoms whose methylene groups may be substituted by oxygen atoms, sulfur atoms, dihydrosilylene, dimethylsilylene, -CH=CH- or -C≡C-, and hydrogen atoms may be substituted by fluorine atoms or chlorine atoms, respectively; rings A1, A2, A3 and A4 each independently represents 1,4-cyclohexylene, 1,4-cyclohexenylene or 1,4-phenylene, carbon atoms in the rings may be replaced by oxygen atoms or nitrogen atoms, and hydrogen atoms in the rings may be substituted by fluorine atoms or chlorine atoms, respectively; and m and n each independently represents 0 or 1.
(2) An alkenyl compound according to (1), wherein at least one of X₁, X₂ and X₃ is -CH₂O- or -OCH₂-.
(3) An alkenyl compound according to (2), wherein Y₁ represents a fluorine atom, a chlorine atom, a cyano group or an alkyl group of 1-20 carbon atoms whose methylene groups may be replaced by oxygen atoms, sulfur atoms, -CH=CH- or -C≡C-.
(4) An alkenyl compound according to (3), wherein m is 0 and n is 1.
(5) An alkenyl compound according to (3), wherein both m and n are 0.
(6) A liquid crystal composition characterized in that it comprises at least one of alkenyl compounds described in any one of (1) to (5).
(7) A liquid crystal composition, characterized in that it comprises as the first component at least one compound selected from the group consisting of the alkenyl compounds described in any one of (1) to (5), and as the second component at least one compound selected from the group consisting of the compounds represented by general formulas (2), (3) and (4): 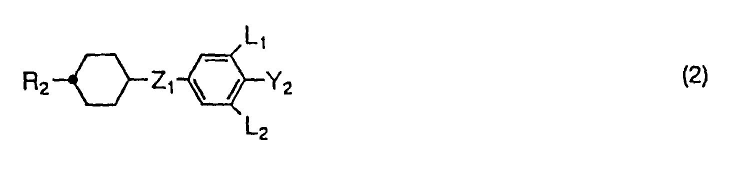 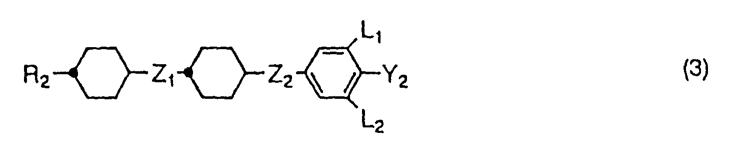 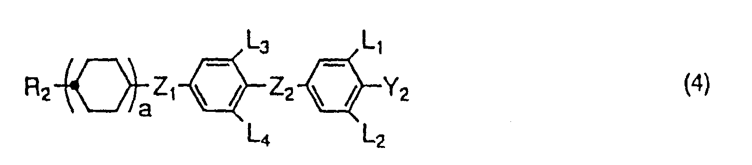 wherein R₂ represents an alkyl group having 1 to 10 carbon atoms; Y₂ represents a fluorine atom, a chlorine atom, OCF₃, OCF₂H, CF₃, CF₂H or CFH₂; L₁, L₂, L₃ and L₄ each independently represents a hydrogen atom or a fluorine atom, Z₁ and Z₂ each independently represents 1,2-ethylene group, -CH=CH- or a single bond, and a is 1 or 2.
(8) A liquid crystal composition, characterized in that it comprises as the first component at least one compound selected from the group consisting of the alkenyl compounds described in any one of (1) to (5), and as the second component at least one compound selected from the group consisting of the compounds represented by general formulas (5), (6), (7), (8) and (9): 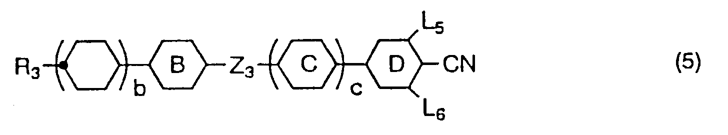 wherein R₃ represents a fluorine atom, an alkyl group having 1 to 10 carbon atoms or an alkenyl group having 2 to 10 carbon atoms; any methylene group (-CH₂-) in the alkyl group or the alkenyl group may be replaced by an oxygen atom, but two or more methylene groups are not continuously replaced by the oxygen atom; ring B represents 1,4-cyclohexylene, 1,4-phenylene or 1,3-dioxane-2,5-diyl; ring C represents a 1,4-cyclohexylene, 1,4-phenylene or pyrimidine-2,5-diyl; ring D represents 1,4-cyclohexylene or 1,4-phenylene; Z₃ represents a 1,2-ethylene group, -COO- or a single bond; L₅ and L₆ each independently represents a hydrogen atom or a fluorine atom; and b and c each independently represents 0 or 1, 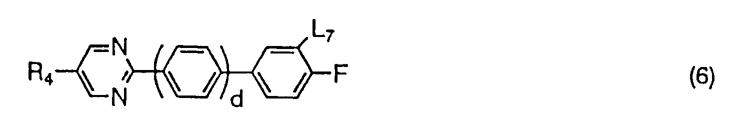 wherein R₄ represents an alkyl group having 1 to 10 carbon atoms, L₇ represents a hydrogen atom or a fluorine atom, and d represents 0 or 1, 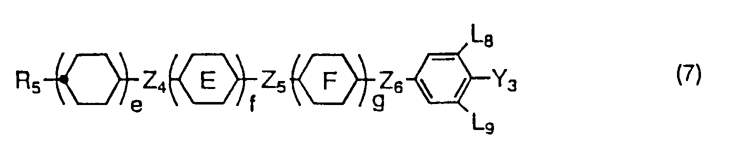 wherein R₅ represents an alkyl group having 1 to 10 carbon atoms; ring E and ring F each independently represents 1,4-cyclohexylene or 1,4-phenylene; Z₄ and Z₅ each independently represents -COO- or a single bond; Z₆ each independently represents -COO- or -C≡C-; L₈ and L₉ each independently represents a hydrogen atom or a fluorine atom; Y₃ represents a fluorine atom, OCF₃, OCF₂H, CF₃, CF₂H or CFH₂; e, f and g each independently represents 0 or 1, 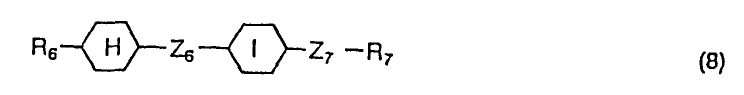 wherein R₆ and R₇ each independently represents an alkyl group having 1 to 10 carbon atoms or an alkenyl group having 2 to 10 carbon atoms, and in any case, any methylene group may be replaced by an oxygen atom, but two or more methylene groups are not continuously replaced by an oxygen atom; ring H represents 1,4-cyclohexylene, 1,4-phenylene or pyrimidine-2,5-diyl; ring I represents 1,4-cyclohexylene or 1,4-phenylene; Z₆ represents -C≡ C-, -COO-, -(CH₂)₂-, -CH=CH-C≡C- or a single bond; Z₇ represents -COO- or a single bond, and 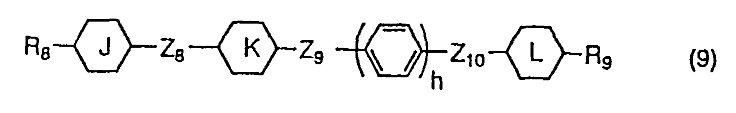 wherein R₈ and R₉ each independently represents an alkyl group having 1 to 10 carbon atoms or an alkenyl group having 2 to 10 carbon atoms, and in any case, any methylene group may be replaced by an oxygen atom, but two or more methylene groups are not continuously replaced by an oxygen atom; ring J represents 1,4-cyclohexylene, 1,4-phenylene or pyrimidine-2,5-diyl; ring K represents 1,4-cyclohexylene, 1,4-phenylene in which one or more hydrogen atoms on the ring may be substituted by a fluorine atom, or pyrimidine-2,5-diyl; ring L represents 1,4-cyclohexylene or 1,4-phenylene; Z₈ and Z₁₀ each independently represents -COO-, a 1,2-ethylene group or a single bond, Z₉ represents -CH=CH-, -C≡C-, -COO- or a single bond; and h represents 0 or 1.
(9) A liquid crystal composition, characterized in that it comprises as the first component at least one compound selected from the group consisting of the alkenyl compounds described in any one of (1) to (5), and as a part of the second component at least one compound selected from the group consisting of the compounds represented by general formulas (2), (3) and (4), and as the other part of the second component at least one compound selected from the group consisting of the compounds represented by general formulas (5), (6), (7), (8) and (9).
(10) A liquid crystal display device constituted by using the liquid crystal composition described in any one of (6) to (9).

The compounds of the present invention are represented by general formula (1) as described in the above.

In the formula, R₁ of the end group represents an alkenyl group of 2-20 carbon atoms, and particularly a trans-1-alkenyl group, a cis-2-alkenyl group and a trans-3-alkenyl group are preferred. As embodied examples, a vinyl group, a 1-propenyl group, a 1-butenyl group, a 1-pentenyl group, a 1-hexenyl group, a 1-heptenyl group, a 1-octenyl group, a 2-propenyl group, a 2-butenyl group, a 2-pentenyl group, a 2-hexenyl group, a 2-heptenyl group, a 2-octenyl group, a 3-butenyl group, a 3-pentenyl group, a 3-hexenyl group, a 3-heptenyl group, a 3-octenyl group, a 1,5-hexadienyl, a 1,5-heptadienyl group, a 4-fluoro-1-butenyl group and a 1,3-butadienyl group can be exemplified.

By suitably selecting the above R₁, K₃₃/K₁₁ having desired magnitude can be attained. For example, when particulary large, middle or somewhat small K₃₃/K₁₁ is desired, it is attained by the R₁ selected from a 3-alkenyl group, a 1-alkenyl group or a 2-alkenyl group.

Then, group Y₁ represents a fluorine atom, a chlorine atom, a bromine atom, an iodine atom, a cyano group or an alkyl group of 1-20 carbon atoms whose methylene groups may be replaced by oxygen atoms, sulfur atoms, silicon atoms, -CH=CH- or -C≡C-, and hydrogen atoms may be substituted by fluorine atoms or chlorine atoms, respectively.

As examples of the above alkyl groups, an alkyl group, an alkoxy group, an alkoxyalkyl group, an alkenyl group, an alkynyl, an alkenyloxy group, an alkynyloxy group, a halogen-substituted alkyl group, a halogenated alkoxy group, a halogen-substituted alkoxy alkyl group, halogen-substituted alkenyl group and halogen-substituted alkynyl group can be exemplified.

More particularly, the following groups can be exemplified: as the alkyl groups each group of methyl, ethyl, propyl, butyl, pentyl, hexyl, heptyl, octyl, nonyl and decyl groups; as the alkoxy groups, each group of methoxy, ethoxy, propoxy, butoxy, pentoxy, hexyloxy, heptyloxy and octyloxy; as alkoxyalkyl groups, each group of methoxymethyl, ethoxymethyl, propoxymethyl, butoxymethyl, methoxyethyl, ethoxyethyl, propoxyethyl, methoxypropyl, ethoxypropyl and propoxypropyl; as the alkenyl group, each group of vinyl, 1-propenyl, 1-butenyl, 1-pentenyl, 3-butenyl and 3-pentenyl; as the alkenyloxy groups, an allyloxy group; as the alkynyl group, each group of ethynyl, 1-propynyl, 1-butynyl, 1-pentynyl, 3-butynyl and 3-pentynyl; as the halogen-substituted alkyl groups, each group of trifluoromethyl, difluoromethyl, difluorochloromethyl, 2,2,2-trifluoro ethyl, 2-fluoroethyl, 3-fluoropropyl, 4-fluorobutyl, 5-fluoropentyl and 3-chloropropyl; as the halogenated alkoxy group, each group of trifluoromethoxy, difluoromethoxy, difluorochloromethoxy, pentafluoroethoxy, 1,1,2,2-tetrafluoroethoxy, heptafluoropropoxy and 1,1,2,3,3,3-hexafluoropropoxy; as the halogen-substituted alkoxy alkyl groups, a trifluoromethoxymethyl group; as the halogen-substituted alkenyl group, each group of 2-fluoroethenyl, 2,2-difluoroethenyl, 1,2,2-trifluoroethenyl, 3-fluoro-1-butenyl and 4-fluoro-1-butenyl; as the halogen-substituted alkynyl groups, a 3,3,3-trifluoro-1-propynyl group.

These groups represented by Y₁ can be appropriately selected and the Δε of compounds are controlled at desired magnitude. For example, when particularly large Δε is desired, Y₁ may be selected from a fluorine atom, a chlorine atom, a cyano group, a halogen-substituted alkyl group, a halogen-substituted alkoxy group, a halogen-substituted alkoxyalkyl group and halogen-substituted alkenyl group; and in the other cases, Y₁ may be selected from an alkyl group, an alkoxy group, an alkoxyalkyl group, an alkenyl group and an alkynyl group, respectively.

Rings A1, A2, A3 and A4 independently represents 1,4-cyclohexylene, 1,4-cyclohexenylene or 1,4-phenylene, carbon atoms in the rings may be substituted by oxygen atoms or nitrogen atoms, and hydrogen atoms may be substituted by fluorine atoms or chlorine atoms, respectively.

As embodied examples that carbon atoms in the rings are replaced by oxygen atoms, there are rings of pyran, 1,3-dioxane and 1,4-dioxane; and as embodied examples that carbon atoms in the rings are replaced by nitrogen atoms, there are rings of pyridine, pyrimidine, pyrazine, pyridazine, triazine, tetrazine and piperidine, respectively.

In these rings, particularly, each ring of 1,4-cyclohexylene, 1,4-cyclohexenylene, 1,4-phenylene, pyridine, pyrimidine and 1,3-dioxane in which hydrogen atoms are substituted by fluorine atoms or chlorine atoms or not substituted, is preferred.

These rings can be appropriately selected and the Δn of compounds are controlled at any desired magnitude. For example, when particularly large Δn is desired, compounds having many benzene rings may be used, because such rings preferably increase parts which have a resonance structure. On the other hand, when small Δn is desired, compounds having many 1,4-cyclohexylene rings or 1,4-cyclohexenylene rings may be used.

The m and n can be appropriately selected to obtain the compounds suitable for controlling the phase transition temperature of liquid crystal compositions. For example, when a particularly high clearing point is desired, a compound of four rings system wherein m and n are 1 may be selected; when a middle clearing point desired, a compound of three rings system wherein m is 1 and n is 0 may be selected; and when a particularly low clearing point is desired, a compound of two rings system wherein both m and n are 0 may be selected.

As described above, the liquid crystalline compounds of the present invention contain many compounds, and all of them have effective characteristics suitable for electric optical display materials, for example, a particularly steep Vₜₕ character, good miscibility to other liquid crystalline compounds, low viscosity, rapid responsibility and Δn and Δε having suitable magnitude. Accordingly, these compounds are useful for components of liquid crystal compositions for STN.

Namely, the liquid crystalline compounds of the present invention have K₃₃/K₁₁ very larger than that of conventional compounds, and the temperature dependence at a low temperature range is very small, so that it is possible particularly to obtain steep Vₜₕ characteristics at a broad temperature range.

Moreover, since the liquid crystalline compounds of the present invention have high solubilities to the other liquid crystalline compounds, even if the liquid crystalline compounds are used for the liquid crystal compositions, the liquid crystal compositions do not damage the nematic phases at low temperatures, for example, at -20°C which Is required for practical use.

Furthermore, since the liquid crystalline compounds of the present invention show low viscosity and its temperature dependence especially at a low temperature is very little, the liquid crystal compositions obtained by using a large amount of the liquid crystalline compounds do not increase in viscosity at the broad temperature range. Accordingly, the liquid crystal compositions having rapid responsibility can be prepared.

Moreover, the liquid crystalline compounds of the present invention have appropriate magnitude of Δn and Δε, and these Δn and Δε may be adjusted by suitably selection of R₁, Y₁, A1, A2, A3, A4, X₁, X₂, X₃, m and n in the general formula (1).

In addition, since the liquid crystalline compounds of the present invention are chemically stable, when the compounds are used as components of the compositions, the resulting compositions have very high specific resistance and voltage holding rates (VHR), and these compounds are very stable to external factors such as ultraviolet light and heating for practical use.

Although the liquid crystalline compounds of the present invention are most preferably used as a component of the liquid crystal compositions for STN, using the excellent characteristics mentioned in the above, the liquid crystalline compounds may be preferably used for the others, for example, liquid crystal display devices of a TN mode, a guest-host mode and a polymer dispersion mode, and liquid crystal compounds for a dynamic scattering mode, active matrix and FLC.

The liquid crystal compositions provided by the present invention may contain only the first component contained at least one compound represented by general formula (1), in addition to the first component, the compositions may be preferably mixed with at least one compound selected from the group consisting of the compounds represented by general formulas (2), (3) and (4) (abbreviated as component 2A hereinafter) and/or the compounds represented by general formulas (5), (6), (7), (8) and (9) (abbreviated as component 2B) as the second component, and further the compositions may be mixed with known compounds as the third component to adjust Vₜₕ, the temperature range of liquid crystal phases, Δn, Δε, viscosity and the like.

In the above component 2A, as embodiments of the compounds represented by general formulas (2), (3) and (4), compounds of (2-1)-(2-15),(3-1)-(3-48) and (4-1)-(4-53) can be preferably exemplified. 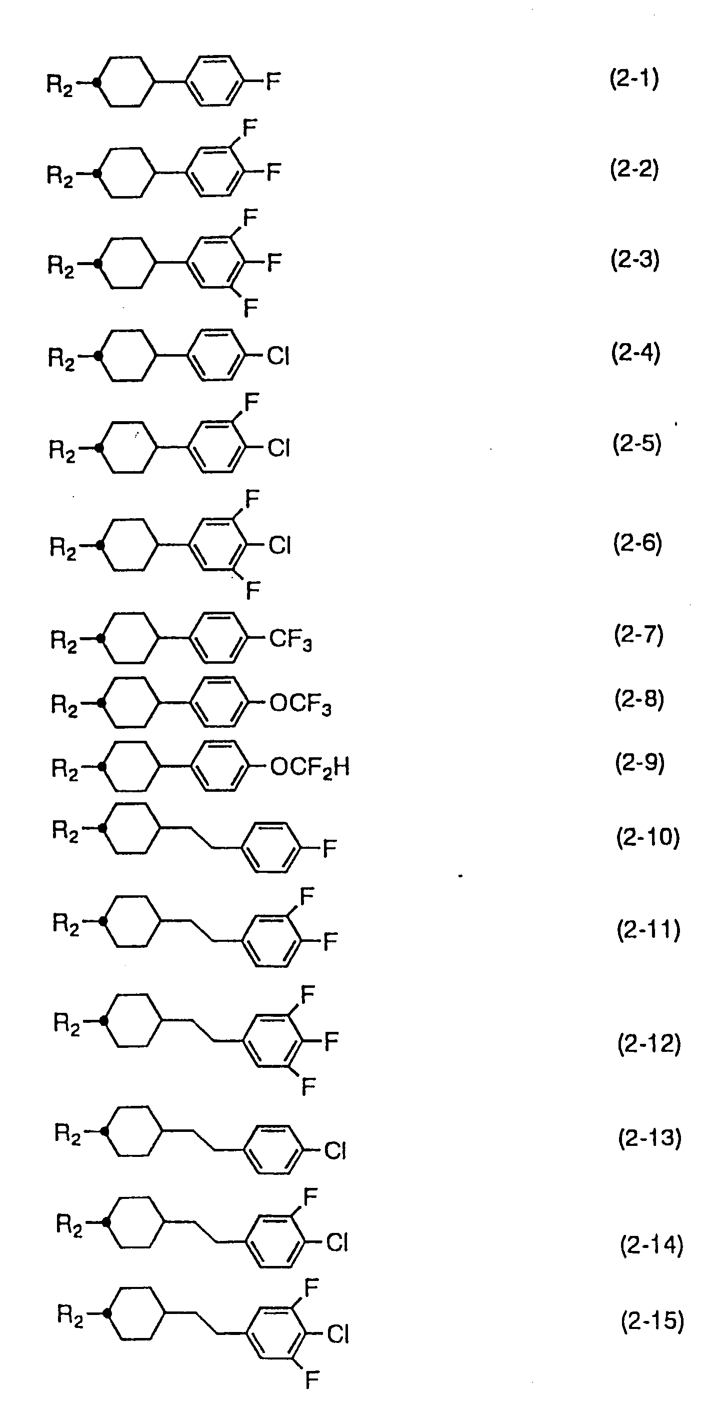 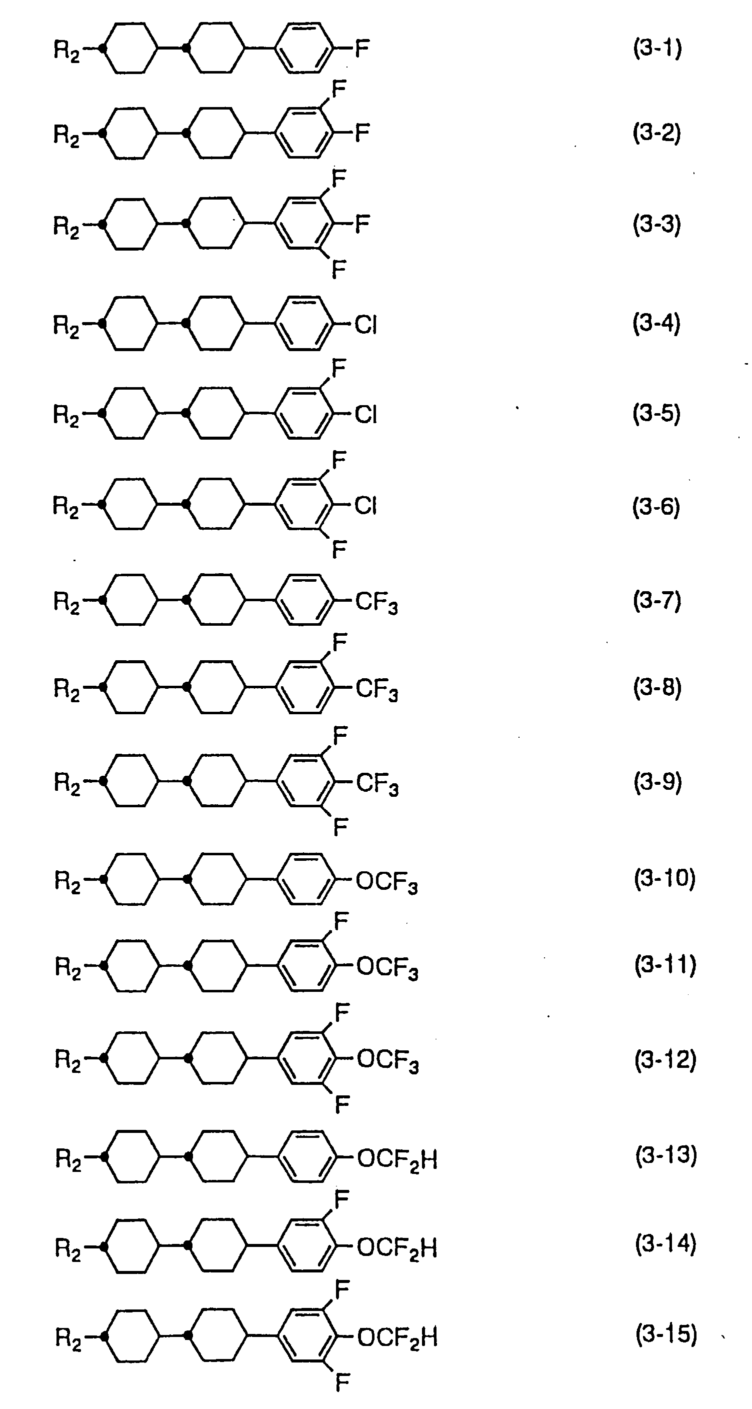 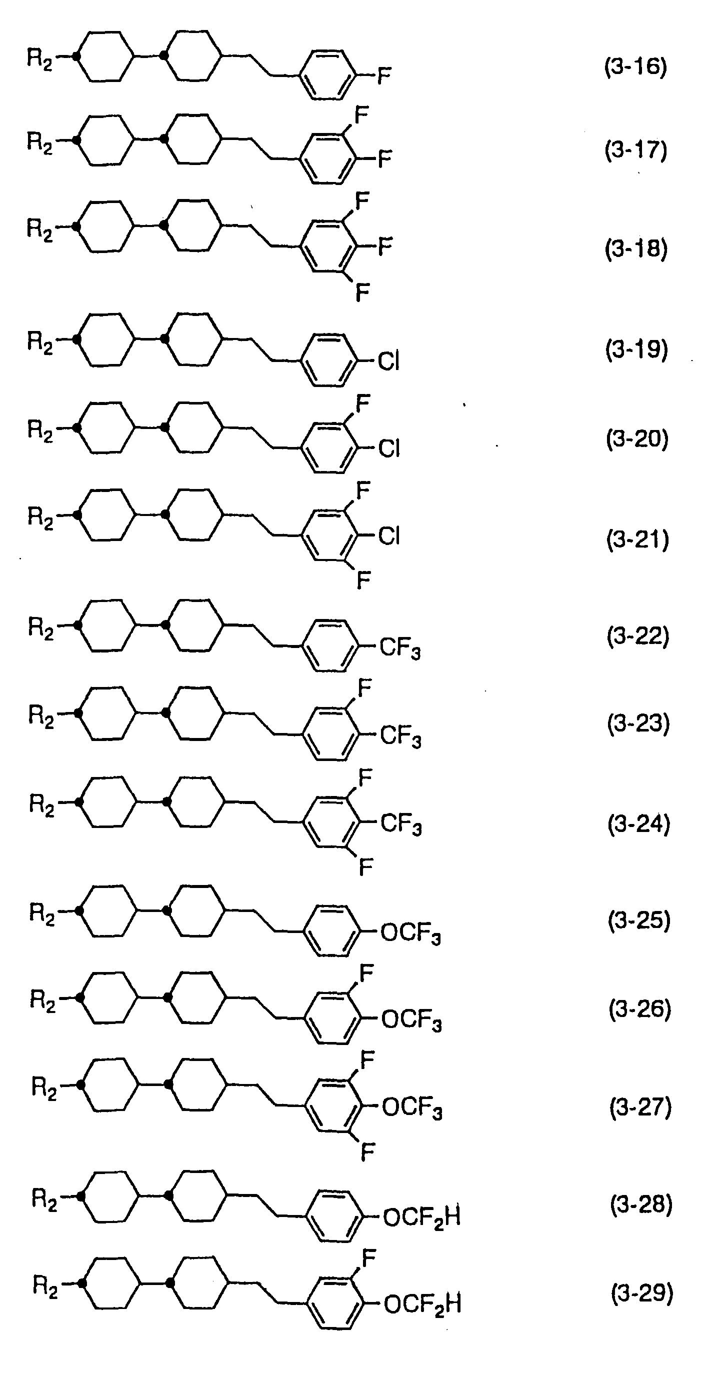 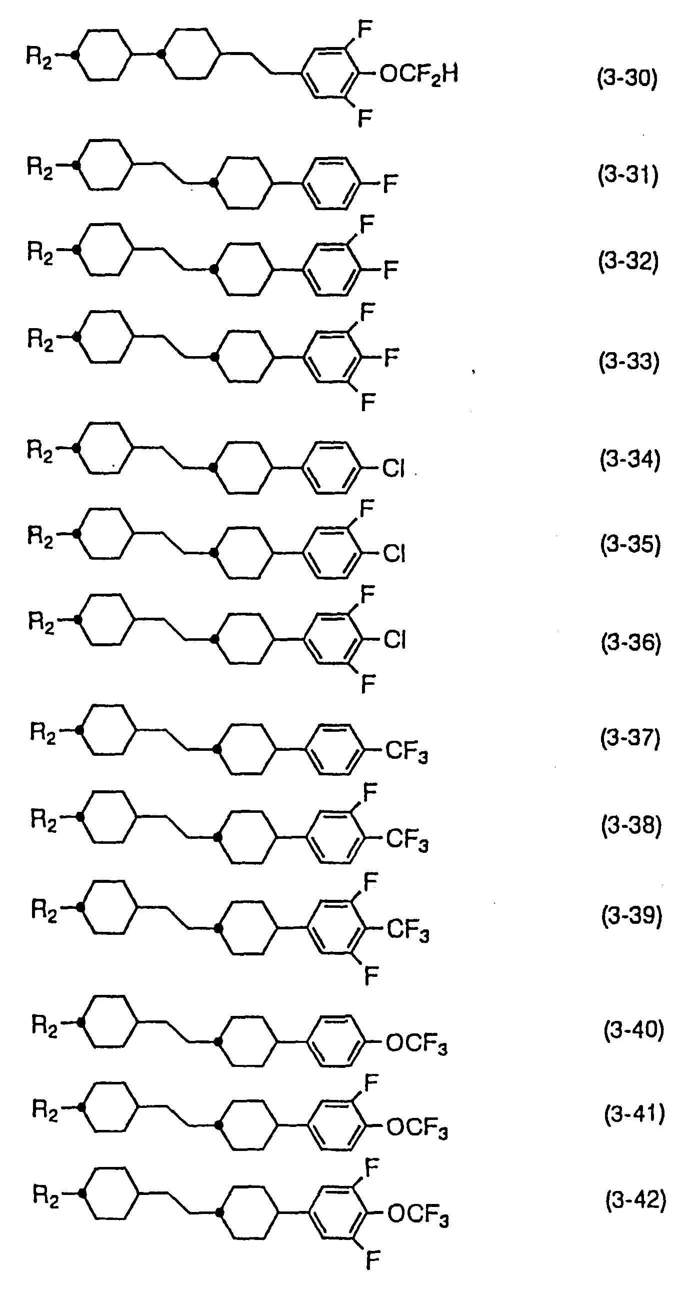 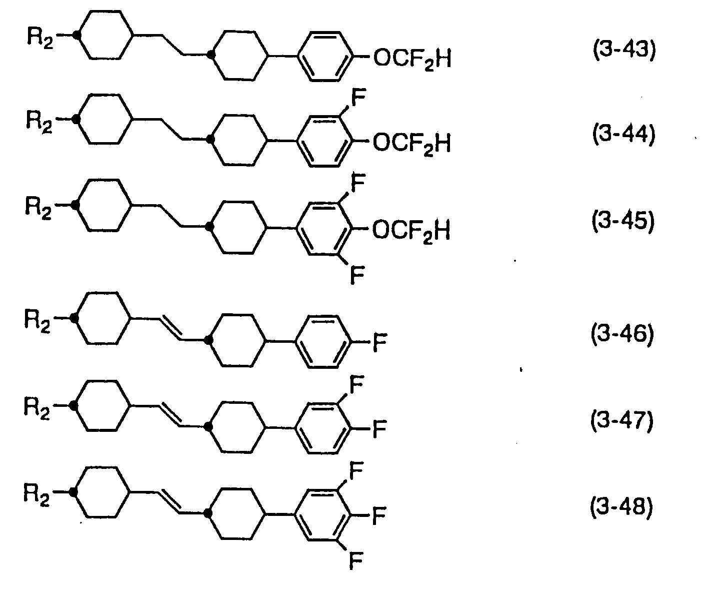 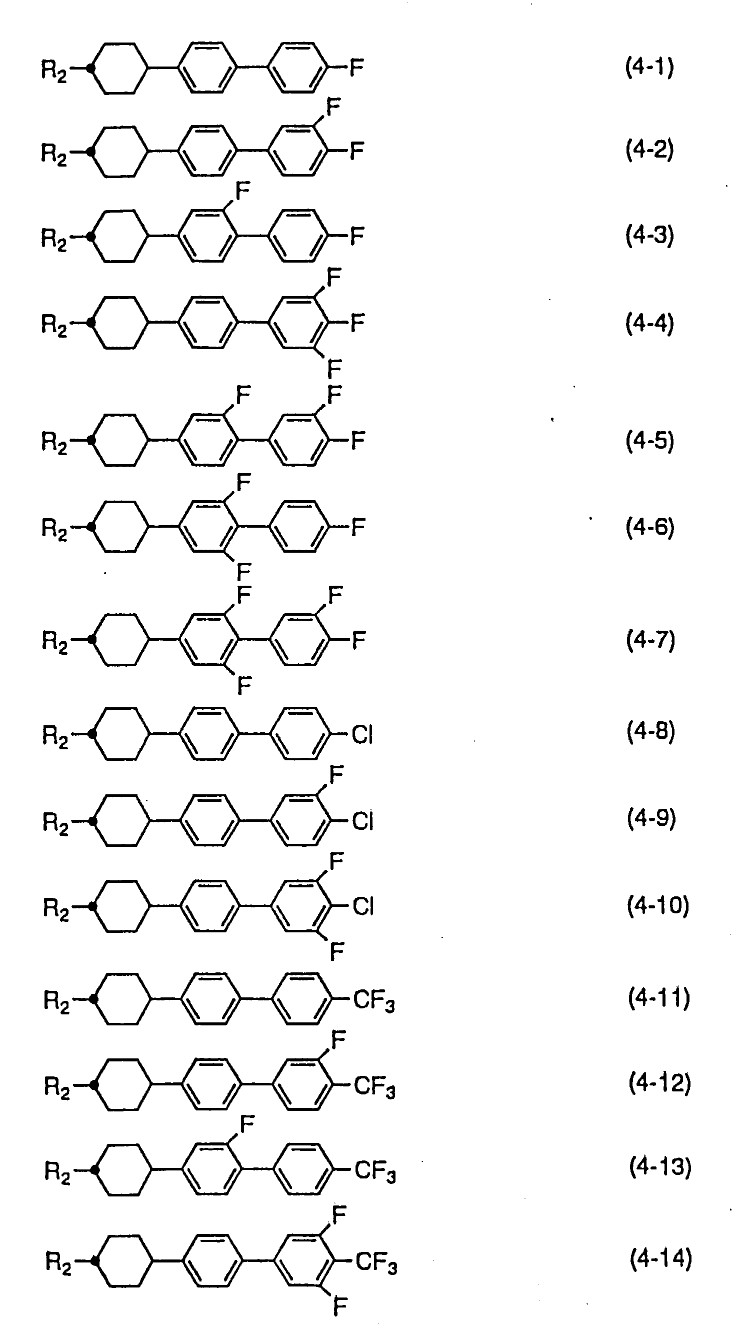 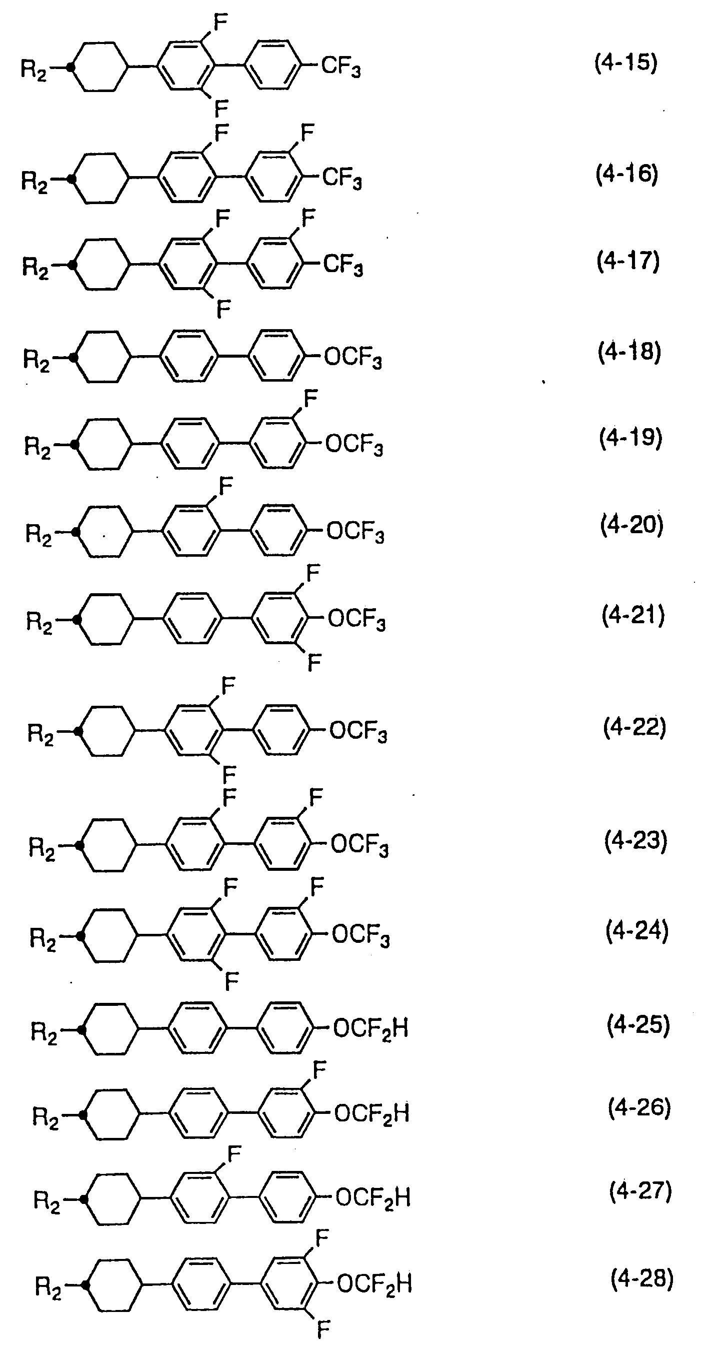 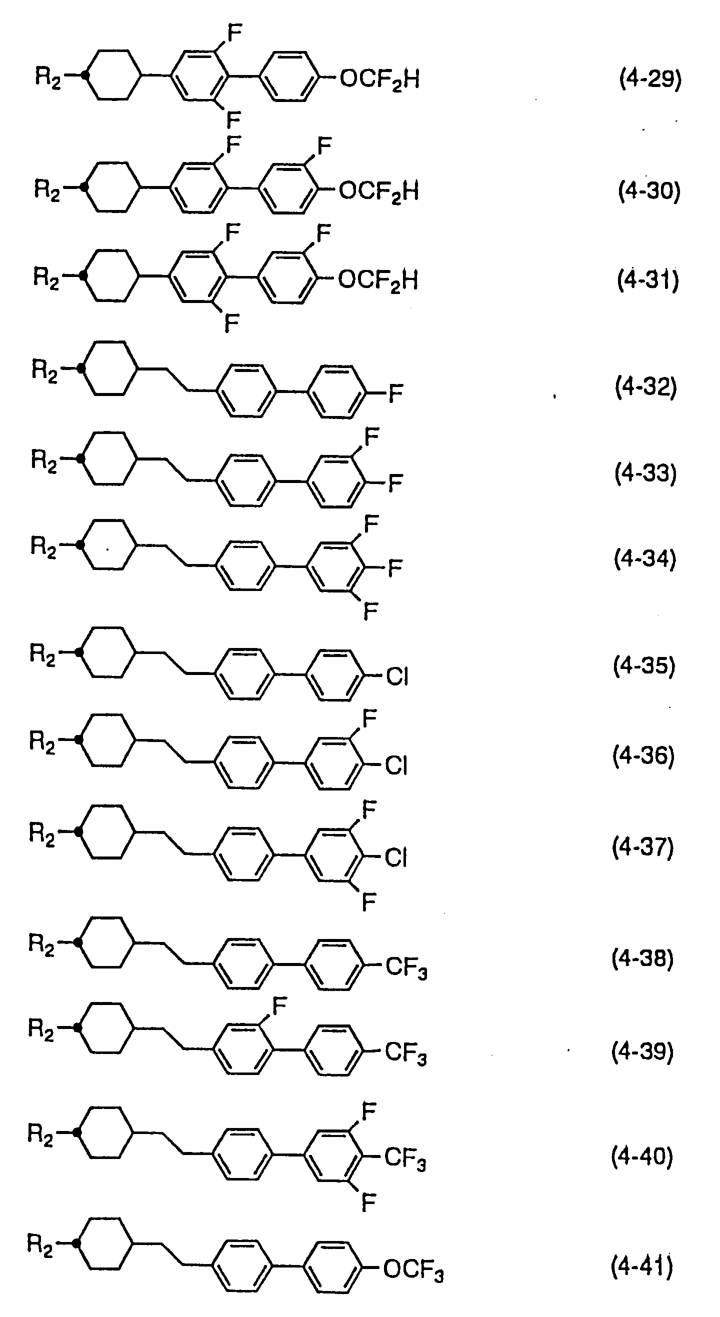 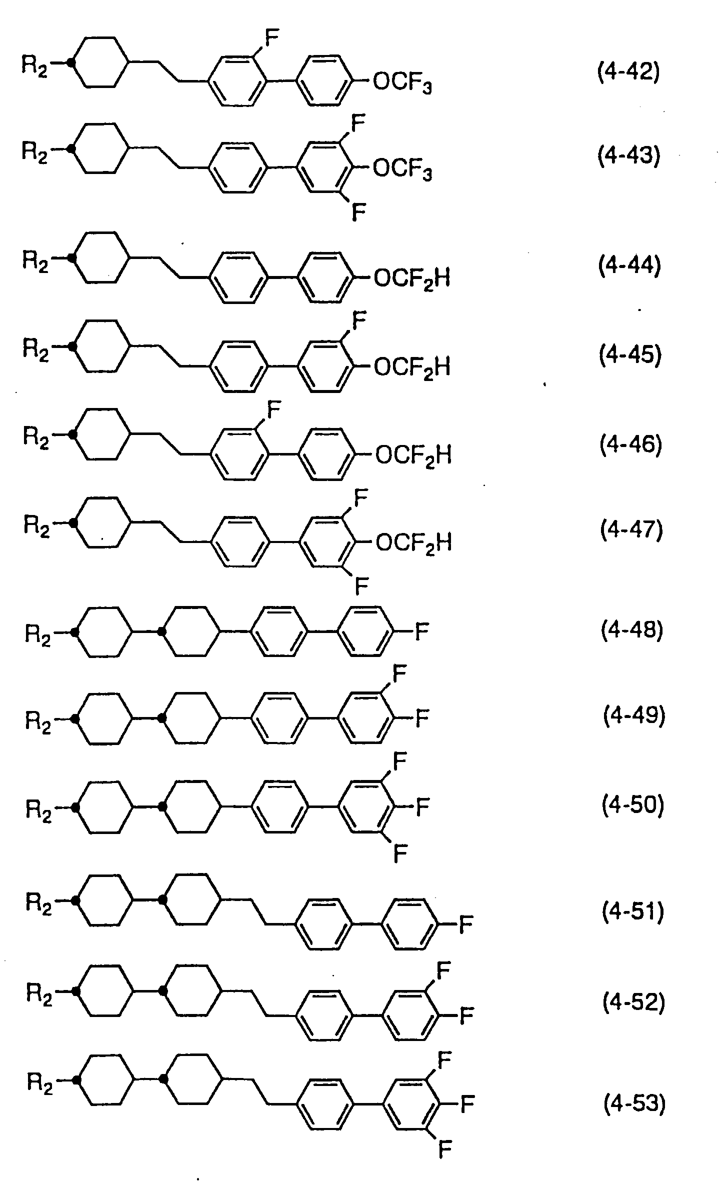 in each formulas, R₂ have the same meaning as the above.

Because compounds represented by these general formulas (2) - (4) show positive Δε, and have stability for temperature and chemical stability, they are necessary to prepare the liquid crystal compositions for TFT in which high reliability such as high voltage holding ratio (VHR) or high specific resistance is required , and these compounds can be used to prepare liquid crystal compositions for display modes of a STN type and common display modes of a TN type.

In case of preparation of display modes of a TFT type, suitable usage of the compound is 1-99% by weight of the total of the liquid crystal composition, preferably 10-97% by weight, and more preferably 40-95% by weight.

Then, in the above component 2B, as embodiments of the compounds represented by general formulas (5), (6) and (7), compounds of (5-1)-(5-24), (6-1) - (6-3) and (7-1)-(7-28) can be preferably exemplified. 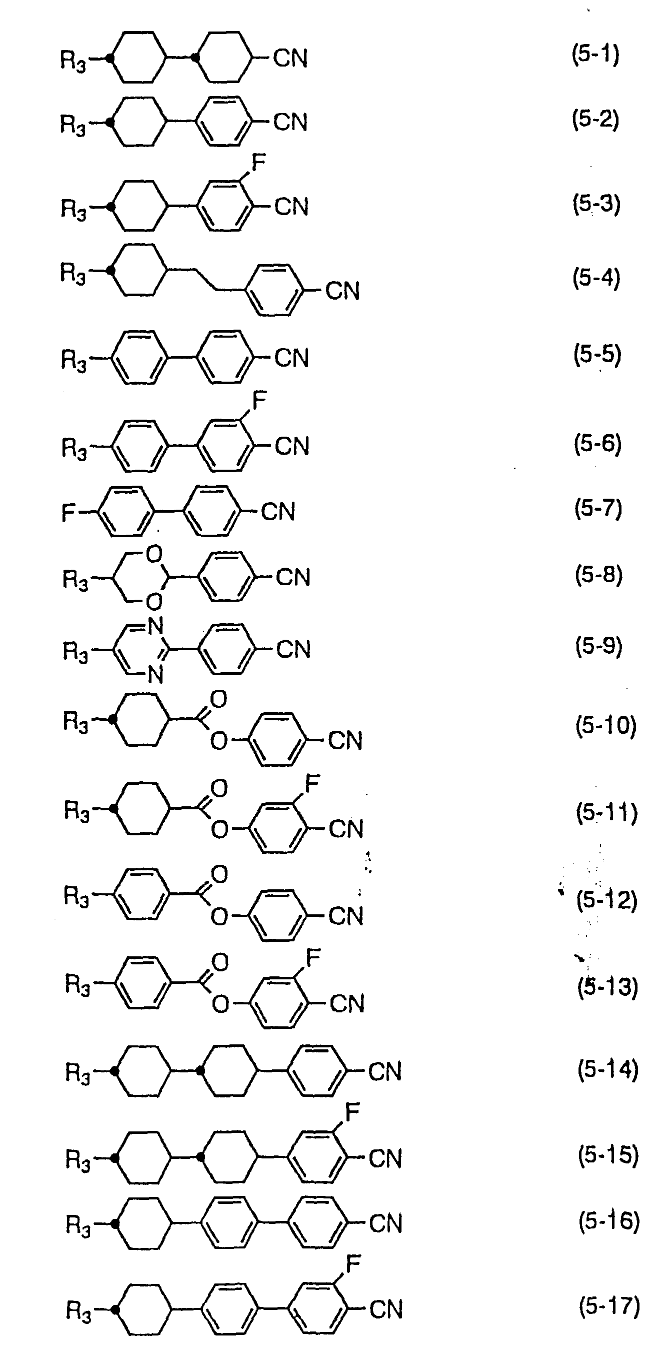 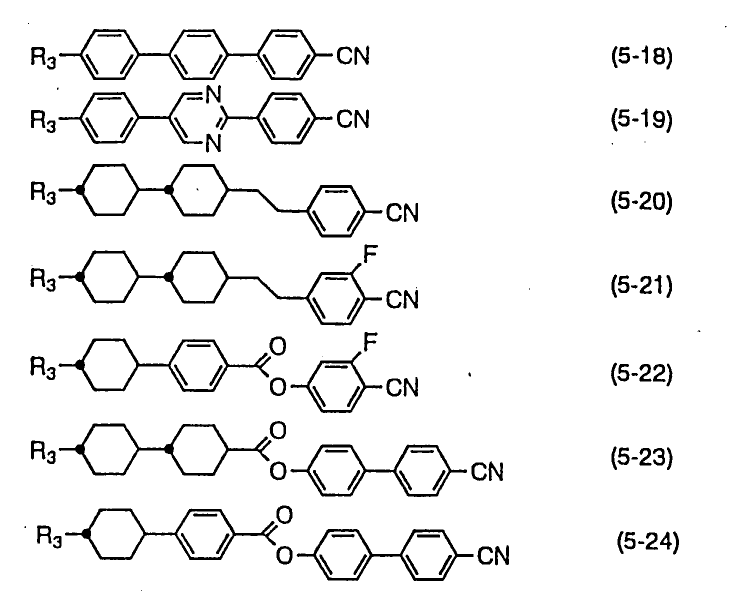 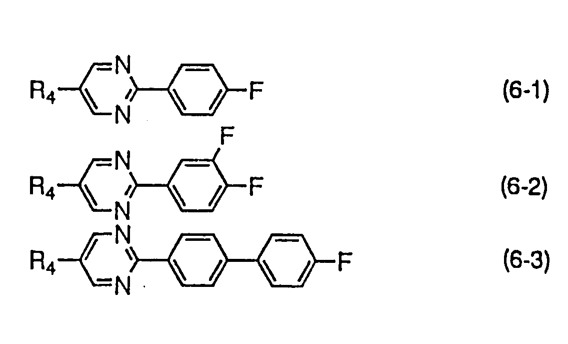 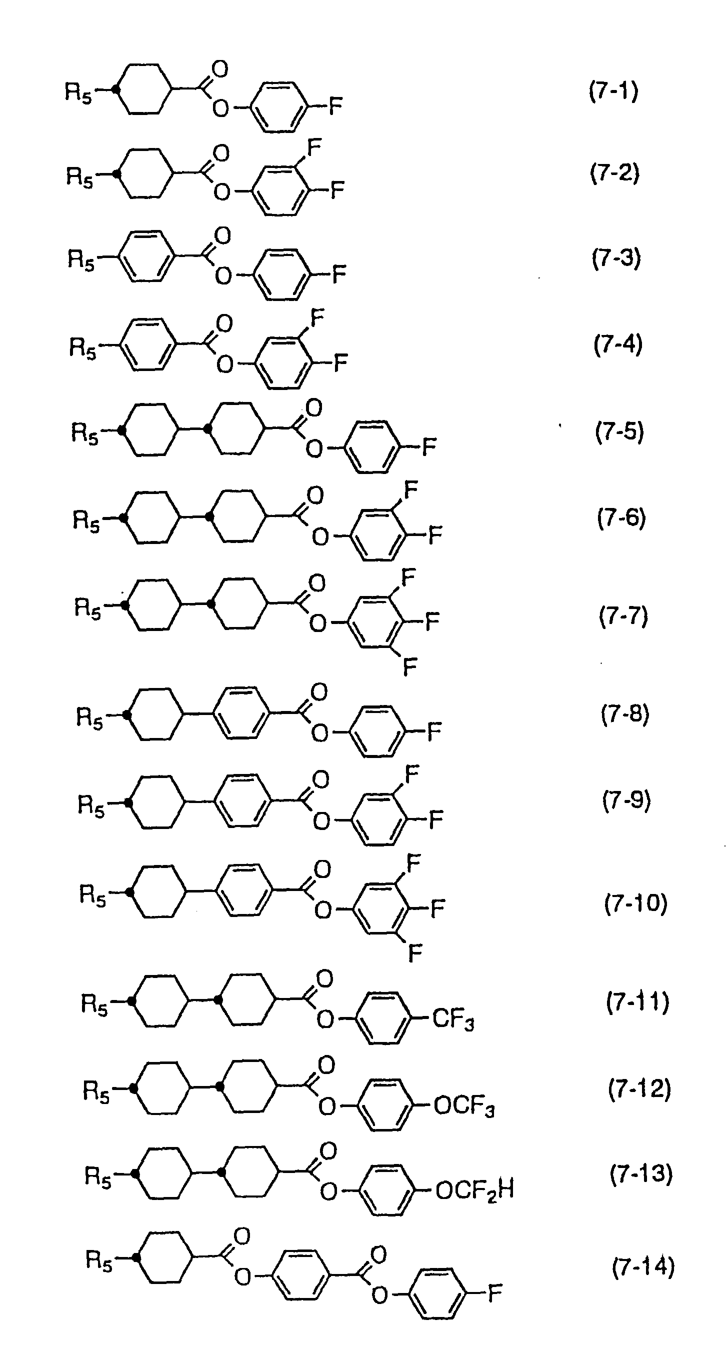 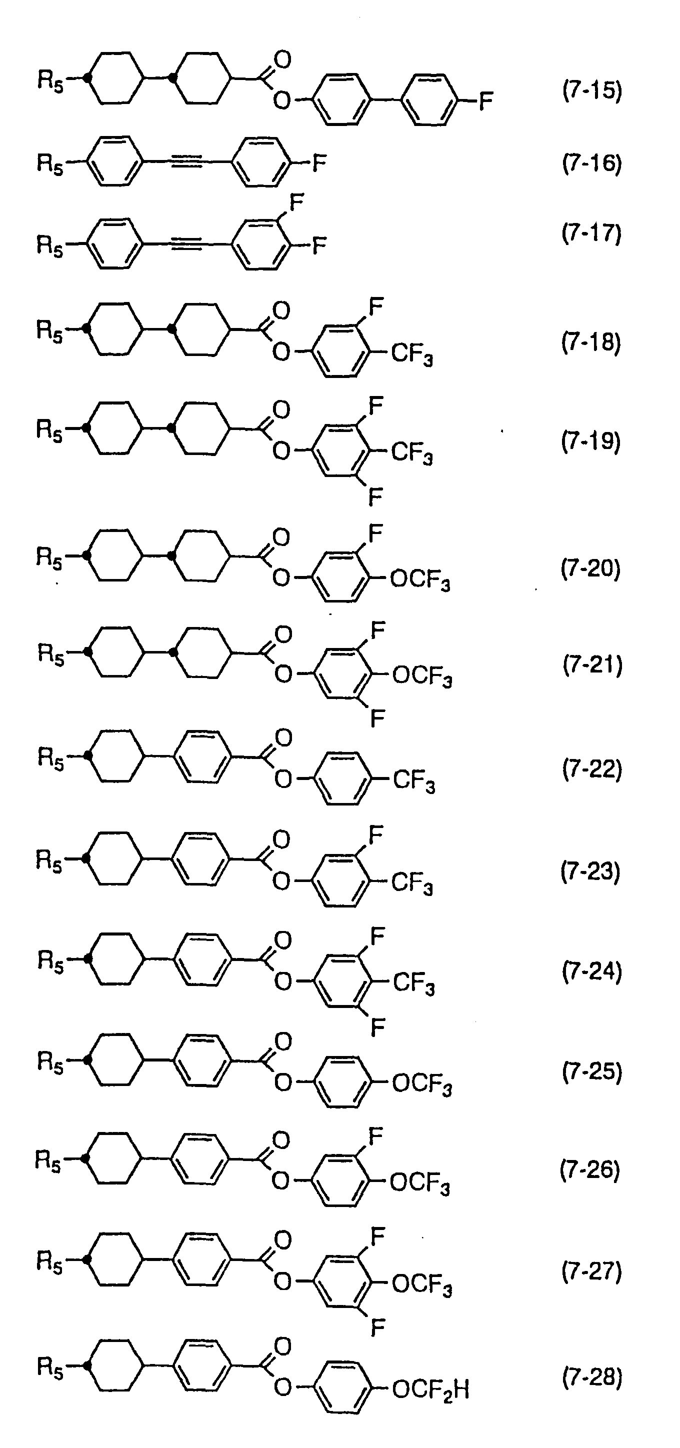 in each formulas, R₃, R₄ and R₅ have the same meaning as the above.

The compounds represented by these general formulas (5) - (7) show positive Δε and its value is large, and these are particularly used as a component of the composition to lower Vth. Addition, these are used to adjust viscosity and Δn, to heighten the clearing point and broaden the nematic range, and to improve steepness of Vₜₕ.

In the above component 2B, as embodiments of the compounds represented by general formulas (8) and (9), compounds of (8-1)-(8-8) and (9-1)-(9-12) can be preferably exemplified. 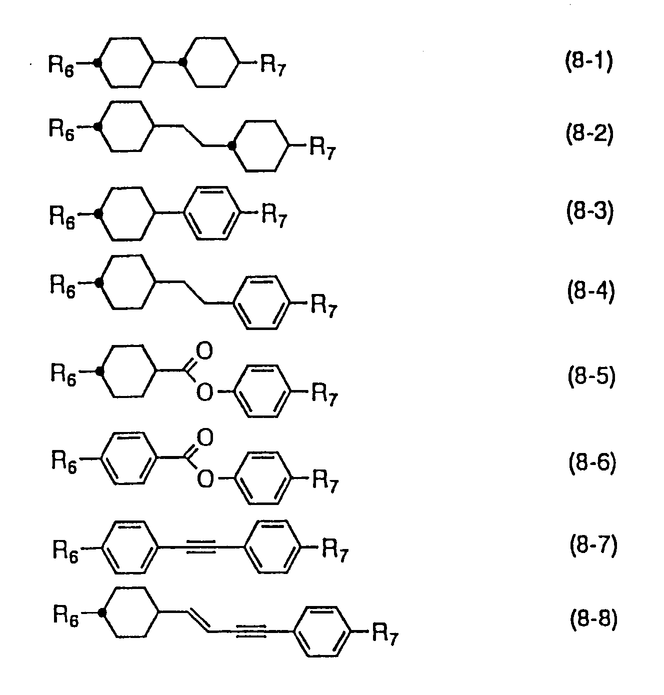 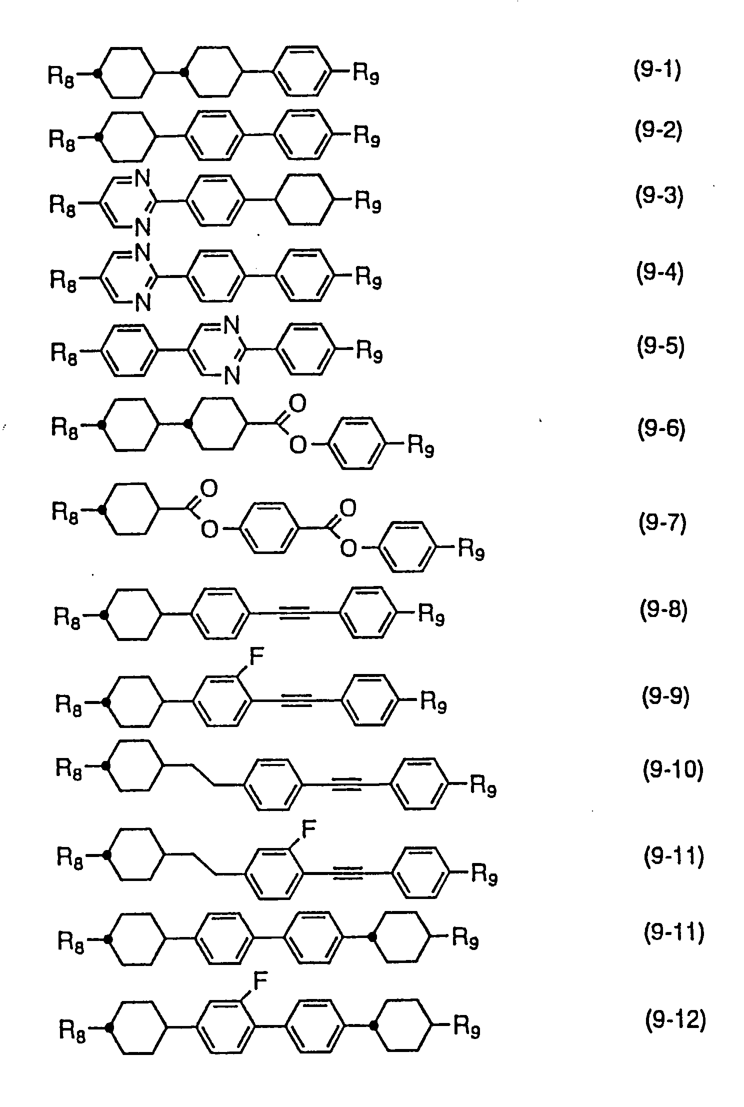 in each formulas, R₆, R₇, R₈ and R₉ have the same meaning as the above.

The compounds represented by these general formulas (8) and (9) show negative or a little positive Δε. In these compounds, the compound represented by general formula (8) is used as a component of the composition to adjust viscosity lowering and Δn, and the compound represented by general formula (9) is used to heighten the clearing point and broaden the nematic range and/or to adjust Δn.

The compounds represented by the above general formulas (5) - (9) are compounds necessary to prepare liquid crystal compositions for a display mode of a STN type or a common display mode of a TN type, and these compounds can be used to prepare aforesaid liquid crystal compositions for TFT.

In case of preparation of liquid crystal composition of common display modes of a TN type or display modes of a STN type, suitable usage of the compound is 1-99% by weight of the total of the liquid crystal composition, preferably 10-97% by weight, and more preferably 40-95% by weight.

The liquid crystal compositions prepared by the above methods can improve the steep Vₜₕ by using for the liquid crystal display devices of the STN mode. Since the compounds of the present invention represented by formula (1) have low viscosity, it is possible to improve the response speed of liquid crystal display devices.

The above-mentioned liquid crystal compositions contain preferably 0.1-99% by weight of at least one kind of liquid crystalline compounds represented by general formula (1) to develop good characteristics.

The liquid crystal compositions of the present invention can be commonly prepared by methods, for example by dissolving components each other at a high temperature, or by mixing each components dissolved in organic solvents and then distilling away the solvents under reduced pressure.

If necessary, suitable additives are added to improve and optimize according to the demand of desired use. Such additives are well-known by the articians concerned in the field, and described in detail in literature and the like. Usually, a chiral dopants and the like are added. The dopants have properties that the spiral structure of liquid crystals is induced to adjust a necessary twisted angle and prevent a reverse twist.

Adding dichroic pigments such as mellocyanine series, styryl series, azo series, azomethine series, azoxy series, quinophthalone series, anthraquinone series and tetrazine series in liquid crystal compositions, the liquid crystal compositions can be used for a guest-host (GH) mode.

The composition of the present invention can be used as a liquid crystal composition for NCAP, which is prepared by a microcapsulation of a nematic liquid crystal, or the composition can be used for a device of polymer-dispersion type liquid crystal display (PDLCD), for example, a device of polymer network liquid crystal display (PNLCD) prepared by forming a threedimentional mesh polymer in liquid crystal, further, for an electrically controlled birefringence (ECB) mode and a DS mode.

The following compositions show examples 1-15 of the liquid crystal compositions of the present invention prepared by the above methods.

In each example of the compositions, the compounds are represented by the symbols representsd in the following Table 1. Left end groups are shown by a-, aO-, aOb-, Va-, aVb- and aVbVd- (a,b and d show an integer of 1 or more), bonding groups are shown by 2, E, T, V, CF2O, OCF2, 1O and O1, ring structures are shown by B, B(F), B(F,F), H, Py, D and Ch, and right end groups are shown by -F, -CL, -C, -CF3, -OCF3, -OCF2H, -w, -Ow and -EMe (w is an integer of 1 or more), respectively. The numbers of the compounds are the same as the numbers represented in after-mentioned examples, and the mixing ratio of each component is representsd by % by weight.

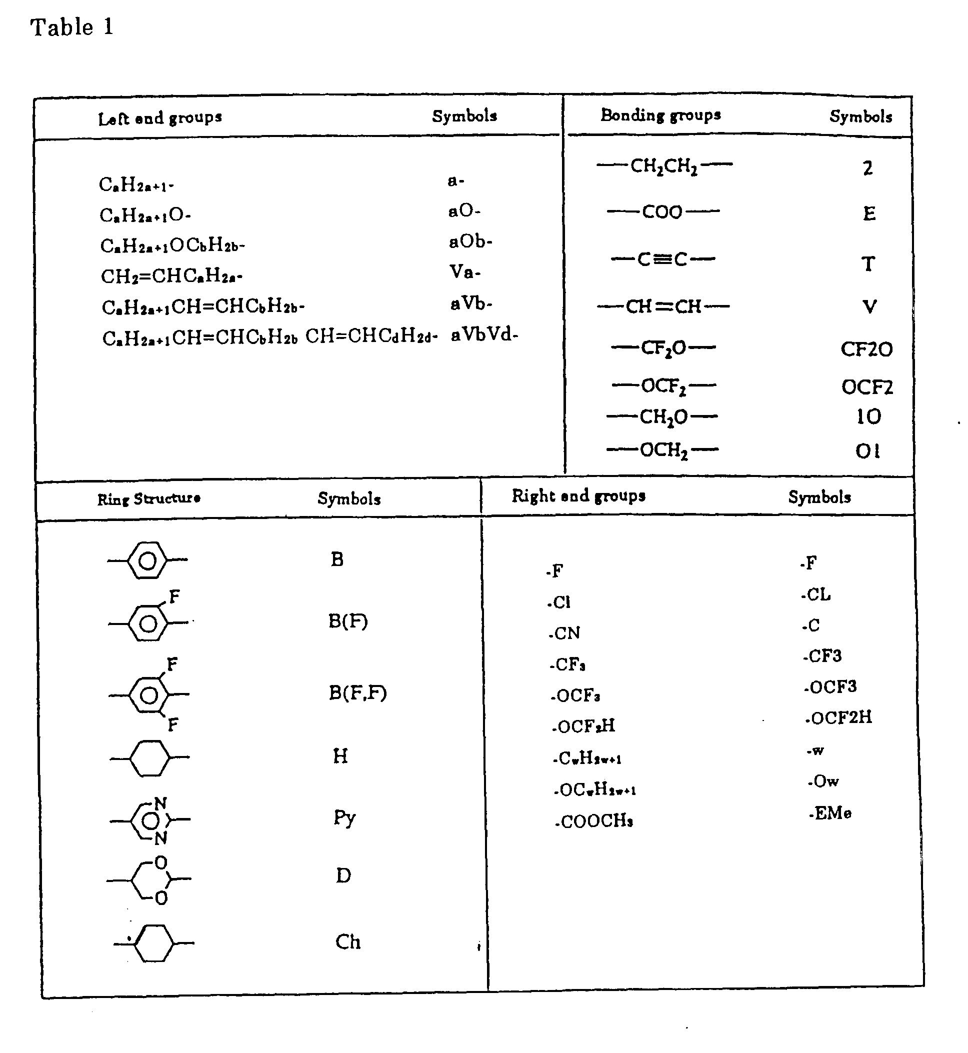

| Composition Example 1 | | |
|---|---|---|
| V-HBO1H-4 | (No. 21) | 8.0% |
| V2-HB-C | | 12.0% |
| 1V2-HB-C | | 11.0% |
| 1V2-BEB(F.F)-C | | 11.0% |
| 2-BTB-1 | | 8.0% |
| 4-BTB-02 | | 8. 0% |
| 5-BTB-O1 | | 6.0% |
| 3-HH-4 | | 3.0% |
| 3-HH-EMe | | 3.0% |
| 3-H2BTB-2 | | 4.0% |
| 3-H2BTB-3 | | 4.0% |
| 3-H2BTB-4 | | 4.0% |
| 3-HB(F)TB-2 | | 6.0% |
| 3-HB(F)TB-3 | | 6.0% |
| 3-HB(F)TB-4 | | 6.0% |

| Composition Example 2 | | |
|---|---|---|
| V-HO1H-3 | (No. 1) | 8.0% |
| V-HBO1H-4 | (No. 21) | 7. 0% |
| V2-HB-C | | 9.0% |
| 1V2-HB-C | | 9.0% |
| 3-HB-C | | 14.0% |
| 1O1-HB-C | | 8.0% |
| 2O1-HB-C | | 4.0% |
| 2-HHB-C | | 5. 0% |
| 3 -HHB-C | | 5. 0% |
| 3-HH-4 | | 4. 0% |
| 1O1-HH-5 | | 8 0% |
| 2-BTB-O1 | | 11.0% |
| 3-HHB-1 | | 8.0% |

| Composition Example 3 | | |
|---|---|---|
| V-HO1H-3 | (No. 1) | 15.0% |
| 3-HB(F)-C | | 5.0% |
| 3O1-BEB(F)-C | | 6.0% |
| V2-HB-C | | 11.0% |
| 2-BTB-O1 | | 5.0% |
| 3-BTB-O1 | | 5.0% |
| 4-BTB-O1 | | 5.0% |
| 4-BTB-O2 | | 5.0% |
| 5-BTB-O1 | | 5.0% |
| 3-H2BTB-2 | | 2.0% |
| 3-H2BTB-3 | | 3.0% |
| 3-H2BTB-4 | | 3.0% |
| 3-HB(F)TB-2 | | 6.0% |
| 3-HB(F)TB-3 | | 6.0% |
| 3-HB(F)TB-4 | | 6.0% |
| 2-PyBH-3 | | 4.0% |
| 3-PyBH-3 | | 4.0% |
| 3-PyBB-2 | | 4.0% |

| Composition Example 4 | | |
|---|---|---|
| V-HBO1H-4 | (No. 21) | 5.0% |
| 1V2-H1OB-C | (No. 2) | 10.0% |
| 3O1-BEB(F)-C | | 6.0% |
| 5O1-BEB(F)-C | | 5.0% |
| 1V2-BEB(F.F)-C | | 10.0% |
| 2-HHB(F)-C | | 5.0% |
| 3-HHEB-F | | 5.0% |
| 5-HHEB-F | | 5.0% |
| 3-HBEB-F | | 6.0% |
| 3-HHB-F | | 3.0% |
| 3-HB-02 | | 10.0% |
| 3-HH-4 | | 6.0% |
| 3-HHB-3 | | 6.0% |
| 3-HHB-O1 | | 4.0% |
| 3-H2BTB-2 | | 4.0% |
| 3-H2BTB-3 | | 4.0% |
| 3-HEBEB-1 | | 3.0% |
| 3-HEBEB-F | | 3.0% |

| Composition Example 5 | | |
|---|---|---|
| 1V2-H1OB-C | (No. 2) | 6.0% |
| V2-HO1HBB-2 | (No. 141) | 2.0% |
| 5-PyB-F | | 5.0% |
| 3-DB-C | | 6.0% |
| 4-DB-C | | 10.0% |
| 2-BEB-C | | 6.0% |
| 3-BEB-C | | 8.0% |
| 3-HHEBB-C | | 3.0% |
| 5-HBEBB-C | | 3.0% |
| 3-PyB(F)-F | | 6.0% |
| 3-HEB-04 | | 11.0% |
| 4-HEB-02 | | 6.0% |
| 5-HEB-O1 | | 6.0% |
| 3-HEB-O2 | | 5.0% |
| 5-HEB-1 | | 4.0% |
| 4-HEB-4 | | 5.0% |
| 10-BEB-2 | | 2.0% |
| 3-HHB-1 | | 6.0% |

| Composition Example 6 | | |
|---|---|---|
| V-HBO1H-4 | (No. 21) | 6.0% |
| V-HO1H-3 | (No. 1) | 6.0% |
| V2-HO1HBB-2 | (No. 141) | 3.0% |
| 5-BB-C | | 7. 0% |
| 7-HEB-F | | 3.0% |
| 3-PyBB-F | | 4. 0% |
| 3-HB-O2 | | 10.0% |
| 3-PyB-4 | | 3.0% |
| 4-PyB-4 | | 3.0% |
| 6-PyB-4 | | 3.0% |
| 3-PyB-5 | | 3.0% |
| 4-PyB-5 | | 3.0% |
| 6-PyB-5 | | 3.0% |
| 6-PyB-O5 | | 4.0% |
| 6-PyB-O6 | | 4.0% |
| 6-PyB-O7 | | 4.0% |
| 6-PyB-O8 | | 4.0% |
| 2-HHB-1 | | 4.0% |
| 3-HHB-1 | | 8.0% |
| 3-HHB-3 | | 10.0% |
| 3-HHB-O1 | | 5.0% |

| Composition Example 7 | | |
|---|---|---|
| V-HBO1H-4 | (No. 21) | 10.0% |
| 3-H2HB(F, F)-F | | 8.0% |
| 5-H2HB(F, F)-F | | 8.0% |
| 3-HHB(F, F)-F | | 8.0% |
| 4-HHB(F, F)-F | | 6.0% |
| 3-HH2B(F, F)-F | | 10.0% |
| 5-HH2B (F, F)-F | | 8. 0% |
| 3-HBB (F, F)-F | | 14.0% |
| 5-HBB(F, F)-F | | 10.0% |
| 3-HHEB(F, F)-F | | 6.0% |
| 4-HHEB(F, F)-F | | 3.0% |
| 3-HBEB(F, F)-F | | 3.0% |
| 3-HHBB (F, F)-F | | 3. 0% |
| 3-HH2BB(F, F)-F | | 3.0% |

| Composition Example 8 | | |
|---|---|---|
| V-HO 1 H-3 | (No. 1) | 8. 0% |
| V-HBO1H-4 | (No. 21) | 5.0% |
| 7-HB(F)-F | | 14.0% |
| 2-HHB(F)-F | | 10.0% |
| 3-HHB(F)-F | | 10.0% |
| 5-HHB(F)-F | | 10.0% |
| 2-H2HB (F) -F | | 6. 0% |
| 3-H2HB(F)-F | | 3.0% |
| 5-H2HB(F)-F | | 6.0% |
| 2-HBB (F) -F | | 7. 0% |
| 3-HBB(F)-F | | 7.0% |
| 5-HBB(F)-F | | 14.0% |

| Composition Example 9 | | |
|---|---|---|
| V-HO1H-3 | (No. 1) | 7.0% |
| V-HO1HVH-3 | (No. 115) | 5.0% |
| V2-HO1HBB-2 | (No. 141) | 3.0% |
| 5-HB-CL | | 7.0% |
| 7-HB(F, F)-F | | 10.0% |
| 2-HBB(F)-F | | 7.0% |
| 3-HBB(F)-F | | 7.0% |
| 5-HBB(F)-F | | 14.0% |
| 4-HHB-CL | | 10.0% |
| 5-HHB-CL | | 5.0% |
| 3-HBB(F, F)-F | | 11.0% |
| 5-HBB(F, F)-F | | 9.0% |
| 3-HB (F) VB-2 | | 5. 0% |

| Composition Example 10 | | |
|---|---|---|
| V-HO1H-3 | (No. 1) | 10.0% |
| 5-H2B(F)-F | | 5.0% |
| 2-HHB(F)-F | | 10.0% |
| 3-HHB(F)-F | | 10.0% |
| 5-HHB(F)-F | | 10.0% |
| 2-HBB(F)-F | | 5.0% |
| 3-HBB(F)-F | | 5.0% |
| 5-HBB(F)-F | | 10.0% |
| 3-HHB(F, F)-F | | 7.0% |
| 5-HHB(F, F)-F | | 4.0% |
| 3-HH2B (F, F) -F | | 9. 0% |
| 5-HH2B(F, F)-F | | 6.0% |
| 5-H2HB(F, F)-F | | 5.0% |
| 3-HBB-F | | 2.0% |
| 5-HHEBB-F | | 2.0% |

| Composition Example 11 | | |
|---|---|---|
| V-HO1H-3 | (No. 1) | 4.0% |
| V-HBO1H-4 | (No. 21) | 3.0% |
| V2-HO1HBB-2 | (No. 141) | 3.0% |
| 2-HHB(F)-F | | 4.0% |
| 3-HHB(F)-F | | 4.0% |
| 5-HHB(F)-F | | 4.0% |
| 2-HBB(F)-F | | 6. 0% |
| 3-HBB(F)-F | | 6.0% |
| 5-HBB(F)-F | | 12.0% |
| 4-H2BB(F)-F | | 8.0% |
| 5-H2BB(F)-F | | 5.0% |
| 3-H2BB(F, F)-F | | 4.0% |
| 4-H2BB(F, F)-F | | 4.0% |
| 5-H2BB(F, F)-F | | 4.0% |
| 3-HBB(F, F)-F | | 10.0% |
| 5-HBB(F, F)-F | | 7.0% |
| 3-HH2B (F, F) -F | | 5. 0% |
| 5-HH2B(F, F)-F | | 5.0% |
| 1O1-HBBH-3 | | 2.0% |

| Composition Example 12 | | |
|---|---|---|
| V-HBO1H-4 | (No. 21) | 4.0% |
| V-HO1H-3 | (No. 1) | 6.0% |
| 6-HB-F | | 8.0% |
| 7-HB-F | | 8.0% |
| 5-HB-3 | | 5.0% |
| 3-HB-O1 | | 5.0% |
| 3-HHB-OCF3 | | 6.0% |
| 4-HHB-OCF3 | | 5.0% |
| 5-HHB-OCF3 | | 7.0% |
| 3-HH2B-OCF3 | | 3.0% |
| 5-HH2B-OCF3 | | 3.0% |
| 3-HH2B-F | | 3.0% |
| 5-HH2B-F | | 3.0% |
| 3-HBB(F)-F | | 6. 0% |
| 5-HBB(F)-F | | 5.0% |
| 3-HH2B(F)-F | | 7.0% |
| 5-HH2B(F)-F | | 9.0% |
| 3-HB(F)BH-3 | | 3.0% |
| 5-HB(F)BH-3 | | 2.0% |
| 5-HB(F)BH-5 | | 2.0% |

| Composition Example 13 | | |
|---|---|---|
| V-HO1HVH-3 | (No. 115) | 5.0% |
| V2-HHO1B(F)B(F)-CF3 | (No. 168) | 5.0% |
| 5-HB-F | | 7.0% |
| 3-HH-O1 | | 7.0% |
| 3-HH-O3 | | 6.0% |
| 3-HHB-OCHF2 | | 3,0% |
| 5-HHB-OCHF2 | | 4.0% |
| 3-HHB(F, F)-OCHF2 | | 8. 0% |
| 5-HHB(F, F)-OCHF2 | | 8.0% |
| 2-HHB-OCF3 | | 6.0% |
| 3-HHB-OCF3 | | 7.0% |
| 4-HHB-OCF3 | | 6.0% |
| 5-HHB-OCF3 | | 6.0% |
| 3-HH2B(F)-F | | 7.0% |
| 5-HH2B(F)-F | | 9.0% |
| 3-HHEB(F)-F | | 6.0% |

| Composition Example 14 | | |
|---|---|---|
| V-HO1H-3 | (No. 1) | 5.0% |
| 1V2-H1OB-C | (No. 2) | 5.0% |
| V-HB-C | | 10.0% |
| 1V-HB-C | | 5.0% |
| 5-BB-C | | 5.0% |
| 2-HB(F)-C | | 5.0% |
| 4-BB-3 | | 3.0% |
| 3-H2B-02 | | 5.0% |
| 5-H2B-02 | | 5.0% |
| 3-BEB-C | | 5.0% |
| 5-HEB-O1 | | 8.0% |
| 5-HEB-03 | | 8.0% |
| 5-BBB-C | | 5.0% |
| 4-BPyB-C | | 4.0% |
| 4-BPyB-5 | | 4.0% |
| 5-HB2B-4 | | 5.0% |
| 5-HBB2B-3 | | 3.0% |
| 1V-HH-1O1 | | 5.0% |
| 1V2-HBB-3 | | 5.0% |

| Composition Example 15 | | |
|---|---|---|
| V2-HH1OB(F)-C | (No. 32) | 8.0% |
| V2-H1OHB-C | (No. 28) | 8.0% |
| 4-HEB(F)-F | | 8.0% |
| 5-HEB(F)-F | | 8.0% |
| 2-BEB(F)-C | | 5. 0% |
| 3-BEB(F)-C | | 5. 0% |
| 5-BEB(F)-C | | 8. 0% |
| 1O3-HB(F)-C | | 6.0% |
| 3-HHEB(F)-F | | 5. 0% |
| 5-HHEB(F)-F | | 5. 0% |
| 2-HBEB(F)-C | | 5.0% |
| 3-HBEB(F)-C | | 6.0% |
| 5-HBEB(F)-C | | 5.0% |
| 3-HBTB-2 | | 10.0% |
| V2-HH-3 | | 4.0% |
| V2-HHB-1 | | 4.0% |

The compounds of the present invention represented by general formula (1) can be easily prepared by combining appropriately the methods described, for example, in Organic Synthesis, Organic reactions, and Jikkenkagaku Koza (Experiment lectures) and the like. These synthesis examples are shown in the following. In these examples, R₁, Y₁, A1, A2, A3, A4, X₁, X₂, X₃, m and n have the same meaning as the above. MG1 shows a left residue of the mesogen selected from formulas (13) - (15), MG2 shows a right residue of the mesogen selected from formulas (16) - (18) (however, formulas (13) and (18), (14) and (17), and (15) and (18) are selected as a pair), Ra shows a hydrogen atom or an alkyl group of 1-18 carbon atoms, Rb shows a hydrogen atom or an alkyl group of 1-17 carbon atoms, Rc shows a hydrogen atom or an alkyl group of 1-16 carbon atoms, and p shows an integer of 1-3.

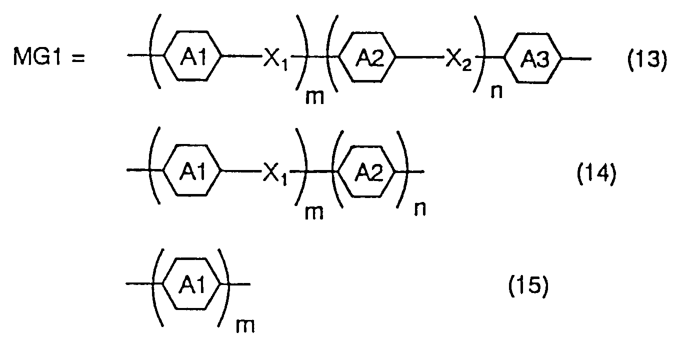

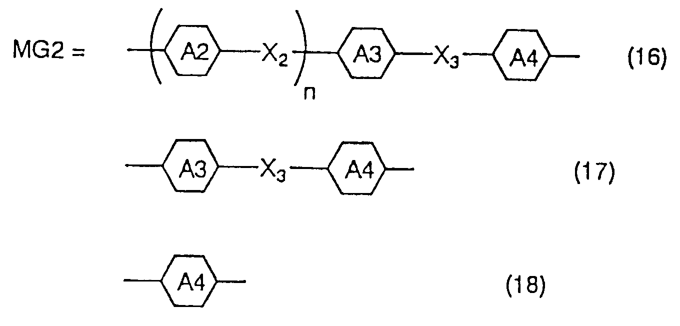

Production of a compound having a methylene oxy group as a central bond group:

Carboxylic acid (19), which is prepared by a common method, is reduced to obtain an alcohol compound, and the compound is halogenated to obtain a halogenated compound (21). The above reduction may be conducted by common methods, by using reducing agents in common use, for example, lithium aluminum hydride (J. Am. Chem. Soc., 87, 1815 (1965)), sodium borohydride (J. Am. Chem. Soc., 93, 2897 (1971)) or a borane/THF complex (J. Am. Chem. Soc., 92, 1637 (1970)). The halogenation can be easily conducted by using bromination agents such as hydrobromic acid (J. Chem. Soc., C, 667 (1971), phosphorus tribromide (J. Am. Chem. Soc., 68, 2513 (1946)) or triphenylphosphino dibromide (Tetrahedron Lett., 913 (1974)), chlorination agents such as thionyl chloride (Org. Synth., II, 136 (1943)) or phosphorus oxychloride (Tetrahedron Lett., 3889 (1967)), or iodination agents such as an iodine/phosphorus type (Org. Synth., II, 322 (1943)) or potassium iodide (Org. Synth., IV, 323 (1963)).

The resulting halogenated compound (21) is treated with an alcohol compound or a phenol compound (23), which are prepared by a common methods, under basic conditions, and the compound of the present invention (1-1) can be easily prepared. The above reaction treatment may be conducted, for example, by the method of Can. J. Chem., 47, 2015 (1969) or Can. J. Chem., 44, 1591 (1966).

Production of a compound having a butylene oxy group as a central bond group:

The compound can be prepared as mentioned above. Namely, carboxylic acid (19) is reacted by adding carbons to obtain a propionic acid derivative (20).

The reaction can be conducted by subjecting a halogenated compound (21) prepared through reduction and halogenation to a repeating reaction operation of cyanidation (Chem. Lett., 471(1973), J. Org. Chem. 26, 2522(1961) and the like), and subsequent hydrolysis (Org. Synth., III, 557 (1955), J. Chem. Soc., 4722 (1962) and the like).

The resulting carboxylic acid derivative (20) is reduced and halogenated to obtain halogenated compound (22) as described above, and the compound is treated with an alcohol compound or a phenol compound (23) as described above to obtain an example of the compound (1-1) of the present invention.

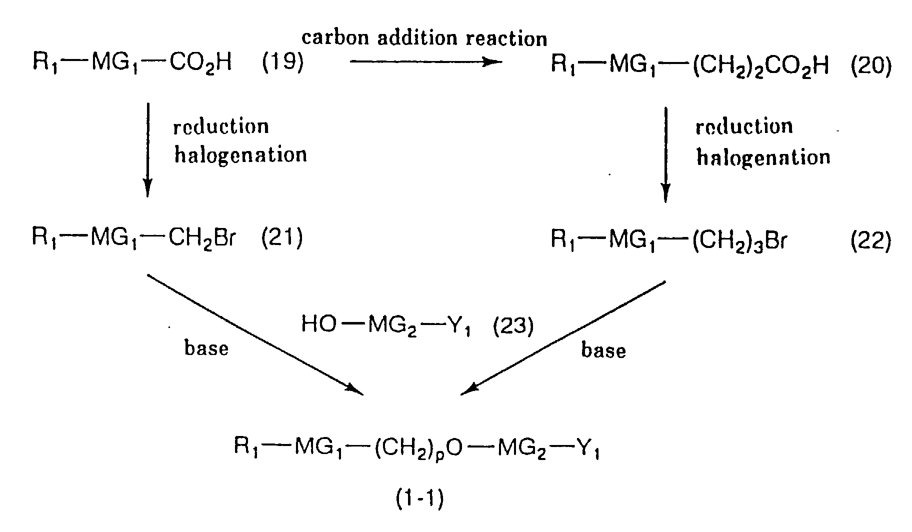

Production of a compound having an oxy methylene group as a central bond group:

As described above, the alcohol compound or phenol compound (24) prepared by a common method, is reacted with halogenated compound (25) or (26) under basic conditions to obtain an example of the compound (1-2) of the present invention.

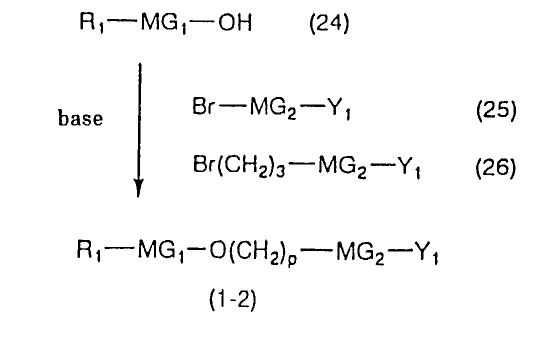

Still more, in the compound (1) of the present invention, an alkenyl position of R₁ is constructed by double bond-forming reaction such as Wittig reaction (J. Am. Chem. Soc., 97, 4327 (1975), J. Chem. Soc., C, 1984 (1968)).

As an embodiment, aldehyde (27) of a starting material, which is prepared by a well-known method, is reacted with a phosphonium salt (28) under basic conditions to obtain a compound (1-3) having an alkenyl group at the primary position. Otherwise, the above aldehyde (27) is treated by a reaction process for increasing carbons, and the resulting aldehyde (30) is reacted with a phosphonium salt (31) as described above to obtain a compound (1-4) having an alkenyl group at the secondary position. The above aldehyde (30) is further treated by a reaction process for increasing carbons, and the resulting aldehyde (33) is reacted with a phosphonium salt (34) as described above to obtain a compound (1-5) having an alkenyl group at the third position. As an base for satisfying the above-mentioned basic conditions, preferably, potassium-t-butoxide, butyllithium and sodium hydride can be exemplified.

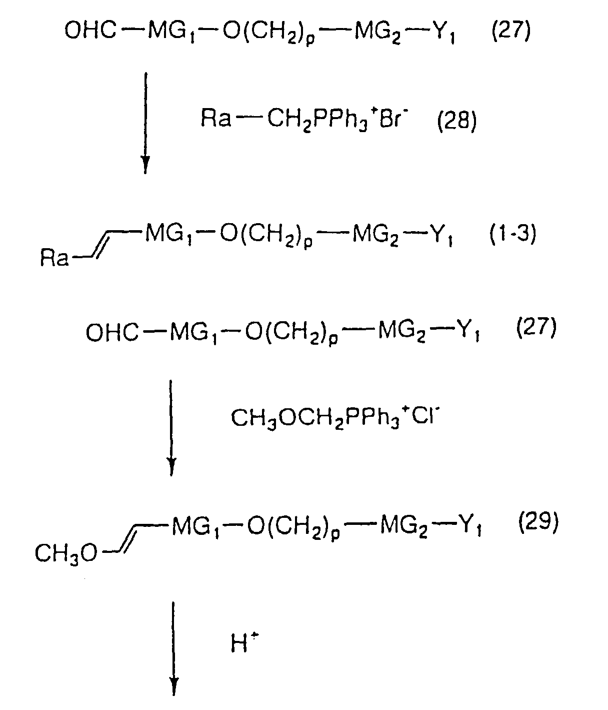

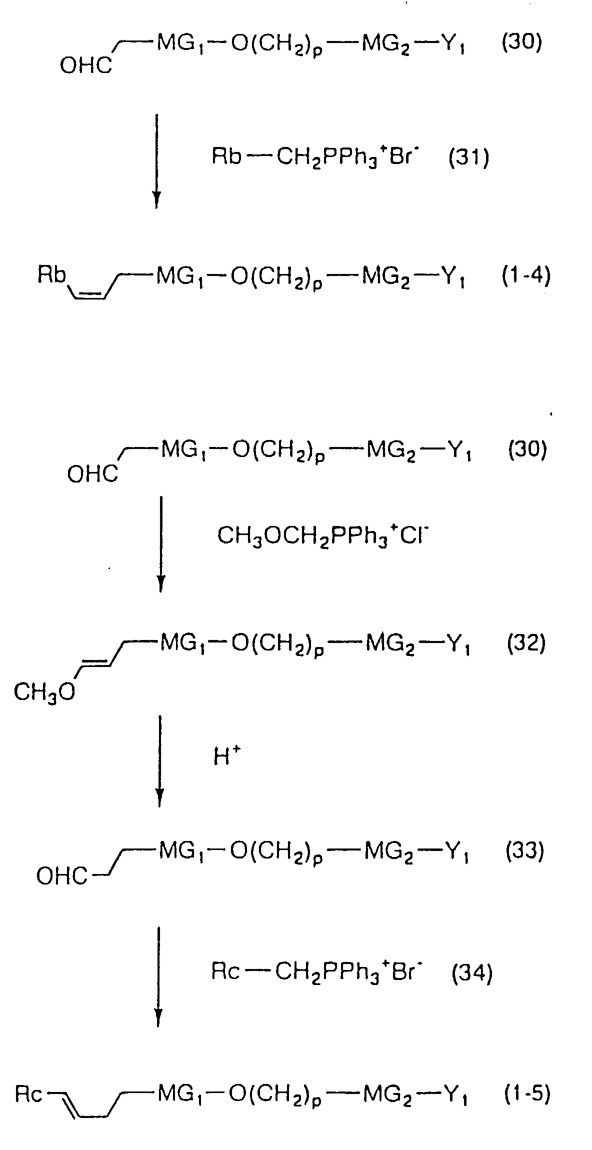

In the compounds prepared as described above, when the double-bond position of the alkenyl group in R₁ is cis form, the cis form is preferably changed to trans form by the method described in Japanese Patent Application Unexamined Laid-open No. 61-83136.

### Best Mode for Carrying Out the Invention

The following examples illustrate the present invention more specifically. In each example, C shows a crystal, N shows a nematic phase, S shows a smectic phase, I shows an isotropic liquid, and the unit of phase transition temperature is °C.

### Example 1

### Preparation of 4-vinyl-1-( (4-propyl-cyclohexyl) methyloxy)cyclohexane (in formula (1), R₁ is a vinyl group, Y₁ is a propyl group, both ring A3 and ring A4 are 1,4-cyclohexylene, both X₁ and X₂, are single bonds, X₃ is a oxymethylene group, and both m and n are 0 (No.1))

Cyclohexane dione monoethyleneketal (1.0 mol) and ethanol 500 ml were mixed. To the mixture, sodium borohydride (0.3 mol) was added dropwise at a temperature of 10°C or less and stirred for 30 minutes. 6 mol hydrochloric acid of 50 ml and then water of 500 ml were added to the mixture at the same temperature. The resulting liquid was extracted three times with 300 ml of diethylether. Obtained organic phase was dried over anhydrous magnesium sulfate, and the solvent was removed under reduced pressure. The residue was recrystallized from pentane to obtain 4-hydroxy-7,10-dioxy-spiro[5,4]decane (0.81 mol, yield 81%).

To the resulting compound 0.80 mol, THF 100 ml and DMSO 200 ml were added. Sodium hydride (about 0.88 mol), which is sufficiently washed with heptane, was slowly added to the mixture and stirred about 1 hour until hydrogen gas was not produced. To the mixture, a solution of 4-propylcyclohexyl methyl bromide (0.8 mol) in THF 100 ml was added, and stirred for 5 hours at room temperature. Under ice cooling, water 200 ml and toluene 200 ml were added, and the mixture was thoroughly stirred and allowed to stand. Separated organic phase was well-washed and dried over anhydrous magnesium sulfate. The solvent was distilled out under reduced pressure, the residue was purified by column chromatography (elute: toluene) and by recrystallization (solvent: ethanol) to obtain 4-((4-propylcyclohexyl)methyloxy)-7,10-dioxy-spiro[5,4]decan (0.48 mol, yield 59%).

A mixture of the resulting compound 0.2 mol, toluene 100 ml and formic acid 10 ml was refluxed for 1 hour, and cooled to room temperature. Water 100 ml was added, and the mixture was thoroughly stirred and allowed to stand. Separated organic phase was well-washed with water and dried over anhydrous magnesium sulfate. The solvent was removed, and the residue was recrystallized from ethanol to obtain 4-((4-(propylcyclohexyl)methyloxy)cyclohexanone (0.19 mol, 95 %).

A solution 50 ml of the resulting compound 0.1 mol in THF was added dropwise to a red homogeneous solution, which is obtained by adding potassium-t-butoxide (0.12 mol) into a mixture of methoxymethyl triphenylphosphonium chloride (0.12 mol) and THF 50 ml at 0 °C or less and by stirring at room temperature for one hour, and the mixture was stirred for three hours.

To the reaction mixture, water 200 ml and heptane 300 ml were added, the mixture was well-stirred and allowed to stand. Separated organic phase was washed with water, the solvent was removed under reduced pressure, heptane 200 ml was added to the resulting residue, and the mixture was well. shaken. After removing insoluble materials by filtration, the filtrate was concentrated under reduced pressure, purified by column chromatography (elute: heptane) to obtain an enolether (0.062 mol, yield 62%).

A mixture of 6M hydrochloric acid 20 ml and THF 50 ml was stirred with the resulting enolether 0.05 mol for a whole day and night, water 50 ml and toluene 100 ml were added, and the mixture was well-stirred and allowed to stand. Separated organic phase was washed with water and dried over anhydrous magnesium sulfate. The solvent was removed under reduced pressure, the residue was purified by column chromatography (elute: toluene) to obtain 4-((4-propylcyclohexyl)methyloxy)cyclohexane carbaldehyde (0.041 mol, yield 41%).

A solution 20 ml of the compound 0.03 mol in THF was added dropwise to a red homogeneous solution, which is obtained by adding potassium-t-butoxide (0.033 mol) into a mixture of methyl triphenylphosphonium bromide (0.033 mol) and THF 20 ml at 0 °C or less and by stirring at room temperature for 1 hour, and the mixture was stirred for 3 hours.

Water 50 ml and heptane 80 ml were added to the mixture. The mixture was well-stirred and allowed to stand. Separated organic phase was washed with water and the solvent was removed under reduced pressure. To the resulting residue, heptane 100 ml was added, and the mixture was well-shaken. After removing insoluble materials by filtration, the filtrate was concentrated under reduced pressure, and purified by column chromatography (elute: heptane) and by recrystallization (solvent: ethanol), and the title compound (0.028 mol, yield 93%) was obtained. The structure was supported well by the spectral data.
GC-MS: 264(M⁺)

### Example 2

### Preparation of 4-(4-(3-pentenylcyclohexyl)methyloxy)benzonitrile (in formula (1), R₁ is a 3-pentenyl group, Y, is a cyano group, ring A3 is 1,4-cyclohexylene, ring A4 is 1,4-phenylene, both X₁ and X₂, are single bonds, X₃ is a methylene oxy group, and both m and n are 0 (No.2))

Methoxymethyl triphenylphosphonium chloride (0.55 mol) and THF 300 ml were mixed. To the mixture, potassium-t-butoxide (0.55 mol) was added at a temperature of 0 °C or less and stirred for one hour at room temperature, and a red homogeneous solution was obtained. To the solution, a solution 200 ml of cyclohexanedionmonoethyleneketal (0.5 mol) in THF was added dropwise, and the mixture was stirred three hours, further water 500 ml and toluene 500 ml were added. The mixture was well-stirred and allowed to stand. Separated organic phase was washed with water and dried over anhydrous magnesium sulfate, and the solvent was removed under reduced pressure. The residue was purified by column chromatography (elute: heptane) to obtain an enolether (0.46 mol, yield 92%).

A mixture of 6M hydrochloric acid 100 ml and THF 300 ml was stirred with the resulting enolether 0.45 mol for three days at room temperature. After adding diethylether 500 ml, the mixture was well-stirred. Separated organic phase was washed with water, and the solvent was removed under reduced pressure to obtain the residue of white crystals of 4-oxocyclohexanecarboxylic acid (0.35 mol, yield 78%).

A mixture of the compound 0.35 mol, ethanol 300 ml and sulfuric acid 5 ml was refluxed for five hours and cooled to room temperature. Then, water 300 ml and heptane 300 ml were added, and the mixture was well-stirred and allowed to stand. Separated organic phase was washed with water and dried over anhydrous magnesium sulfate, and the solvent was removed under reduced pressure. The residue was purified by column chromatography (elute: toluene) to obtain colorless oil of 4-oxocyclohexanecarboxylic acid ethylester (0.3 mol, yield 86%).

A solution 100 ml of the compound 0.3 mol in THF was added dropwise at a temperature of 10 °C or less to a red yellow homogeneous solution, which is obtained by stirring a mixture comprising of 1,3-dioxane-2-yl ethyl)triphenylphosphonium bromide (0.33 mol), potassium-t-butoxide (0.33 mol) and THF 300 ml for one hour, and the mixture was stirred for three hours. Water 400 ml and toluene 300 ml were added to the reactant and well-stirred. Separated organic phase was washed with water and the solvent was removed under reduced pressure. To the resulting residue, heptane 200 ml was added, and the mixture was well-shaken. After removing insoluble materials by filtration, the filtrate was concentrated under reduced pressure, and purified by column chromatography (elute: heptane). To the resulting purified solution, a mixture of ethanol 200 ml, toluene 100 ml and 10% palladium-carbon 20 g was added, and the mixture was stirred for five hours in an atmosphere of hydrogen.

The catalyst was filtered and removed from the reaction system, the filtrate was concentrated under reduced pressure, and the residue was purified by column chromatography (elute: toluene) to obtain 4-(2-(1,3-dioxy-2-yl)ethyl)cyclohexanecarboxylic acid ethylester (0.21 mol, yield 69%). The compound was a trans/cis mixture and used for the next reaction as it is without separation.

A mixture of the compound 0.2 mol, toluene 100 ml and formic acid 30 ml was refluxed for one hour and cooled, and water 100 ml was added and the mixture was thoroughly stirred. Separated organic phase was washed with water and dried over anhydrous magnesium sulfate, and the solvent was removed under reduced pressure. The resulting residue was purified by column chromatography (elute: toluene) to obtain 4-(3-oxopropyl)cyclohexanecarboxylic acid ethylester (0.19 mol, yield 95%).

A solution 50 ml of the compound 0.15 mol in THF was added dropwise at 10 °C or less to a solution, which was obtained by stirring a mixture of ethyltriphenylphosphoniumbromide (0.17 mol), potassium-t-butoxide (0.17 mol) and THF 70 ml at room temperature for two hours, and the mixture was stirred at room temperature for three hours.

Water 100 ml and toluene 100 ml were added to the reactant and the mixture was well-stirred, and separated organic phase was concentrated under reduced pressure. The residue was purified by column chromatography (elute: heptane) and cis-4-(3-pentenyl)cyclohexanecarboxylic acid (0.11 mol, yield 72%) was obtained.

A mixture of the compound 0.1 mol, sodium carbonate (0.4 mol), meta-chloroperbenzoic acid (0.2 mol) and dichloromethane 300 ml was stirred a whole day and night at room temperature. Toluene 200 ml and water 50 ml were added to the reactant and well-mixed. Separated organic phase was washed three times with 100 ml of a saturated solution of sodium thiosulfate and dried over anhydrous magnesium sulfate, and the solvent was removed under reduced pressure to obtain a crude epoxide (0.1 mol, yield 99%).

A mixture of the compound 0.1 mol, triphenylphosphindibomide (0.2 mol) and toluene 100 ml was refluxed for three hours and cooled, and well-washed with an aqueous saturated solution of sodium bicarbonate. After drying the solution over anhydrous magnesium sulfate, and the solvent was removed under reduced pressure. The resulting residue was purified by column chromatography (elute: toluene), and recrystallized from ethanol and benzene, each two times.

White crystals of erythro-4-(3,4-dibromopentyl)cyclohexanecarboxylic acid ethylester (0.023 mol, yield 23%) was obtained.

A mixture of the compound 0.02 mol, acetic acid 100 ml and zinc (0.16 mol) was stirred at room temperature a whole day and night. Water 100 ml and heptane 70 ml were added to the reactant and the mixture was well-stirred, and allowed to stand. Separated organic phase was thoroughly washed with an aqueous saturated solution of sodium bicarbonate and with water, and dried over anhydrous magnesium sulfate. After removing the solvent under reduced pressure, the residue was purified by column chromatography (elute: heptane) to obtain colorless oil of 4-(3-pentenyl)cyclohexanecarboxylic acid ethylester (0.17 mol, yield 85%).

A mixture of the compound 0.15 mol and toluene 30 ml was cooled to -65 °C, and a solution (about 0.015 mol) of aluminum diisobutylhydride in toluene was added dropwise at the same temperature. After stirring at the same temperature for one hour, methanol 10 ml and 6M hydrochloric acid 30 ml were added, and the temperature was slowly raised to room temperature. Precipitated insolubles were filtered out with Celite (Johns-Manville Company's trade name), and the filtrate was concentrated under reduced pressure. The residue was purified by column chromatography (elute: a mixed solution of toluene and ethyl acetate) to obtain 4-(3-pentenyl)cyclohexyl methanol (0.14 mol, yield 93%).

Carbon tetrabromide (0.15 mol) was added to a mixture of the compound 0.1 mol, triphenylphosphine (0.2 mol) and dichloromethane 20 ml and stirred at room temperature for three hours. Water 50 ml was added to the reactant and thoroughly stirred. Separated organic phase was dried over anhydrous magnesium sulfate. After removing the solvent under reduced pressure, the residue was purified by column chromatography (elute: heptane) to obtain 4-(3-pentenyl)cyclohexylmethyl bromide (0.1 mol, yield 99%).

When production of hydrogen gas was stopped, a solution 20 ml of the compound 0.1 mol in THF was added to the following solution; a solution which is obtained by adding sodium hydride (0.11 mol) to a mixture of 4-cyanophenol, THF 10 ml and DMSO 5 ml, and by stirring at room temperature for two hours; and the mixture was stirred for three hours at 60 °C. Then, after adding water 20 ml under cooling, the solution was well-extracted with heptane 50 ml, and the solvent was removed under reduced pressure. The residue was purified by column chromatography (elute: heptane) and then by recrystallization (solvent: ethanol) to obtain the title compound (0.035 mol, yield 35%). The structure was supported well by the spectral data.
GC-MS: 283(M⁺)

The following compounds (No. 3 - 20) can be prepared according to the above methods of Example 1 and Example 2.

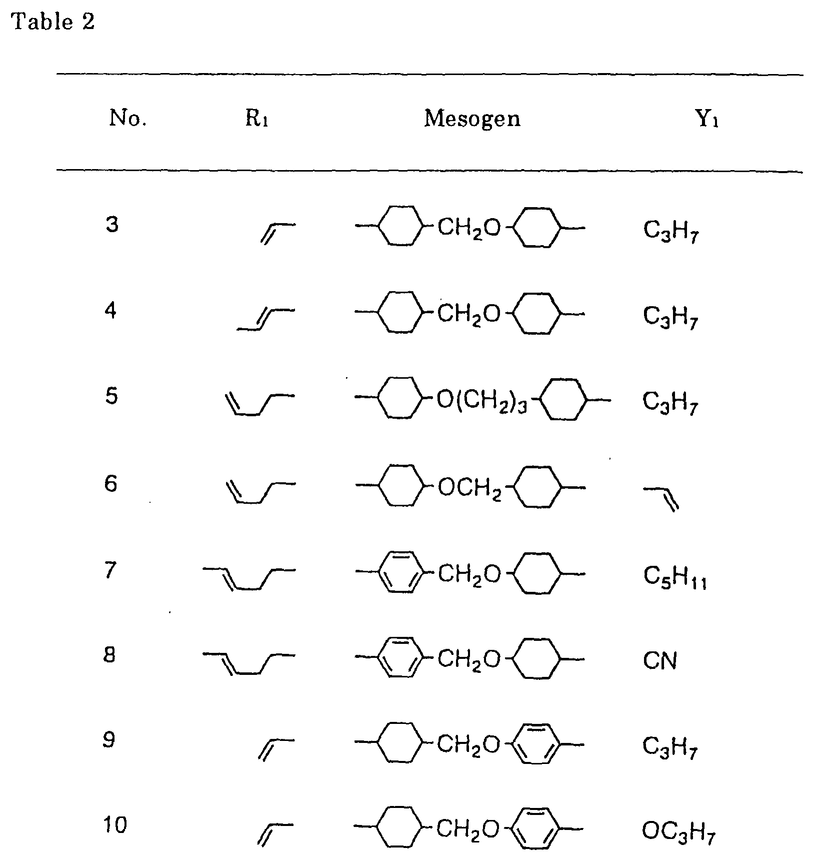

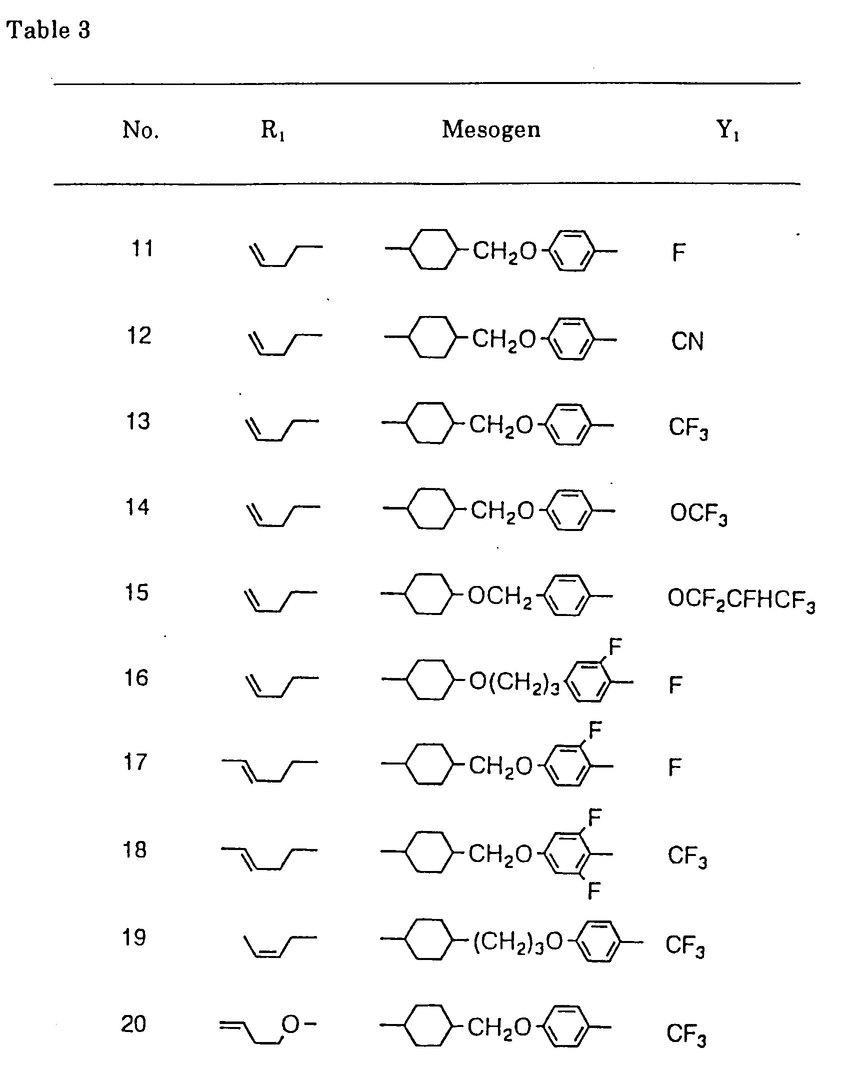

### Example 3

### Preparation of 4-((4-butylcyclohexyl)methyloxy)phenyl-1-vinylcyclohexane (in formula (1), R₁ is a vinyl group, Y₁ is a butyl group, both ring A2 and ring A4 are 1,4-cyclohexylene, ring A3 is 1,4-phenylene, both X₁ and X₂ are covalent bonds, X₃ is a methylene oxy group, and m is 0 and n is 1 (No.21))

A mixture of 4-bromophenol (0.5 mol), 4-butylcyclohexylmethylbromide (0.5 mol), potassium hydroxide (0.55 mol), water 100 ml and ethyl cellosolve (UCC company's trade name) 500 ml was refluxed for eight hours and cooled, toluene 500 ml was added, and the mixture was well-stirred. Separated organic phase was thoroughly washed with water and dried over anhydrous magnesium sulfate, and the solvent was removed under reduced pressure to obtain the residue of colorless oil of 4-(4-butylcyclohexyl)methyloxy -1-bromobenzene (0.5 mol, quantitative yield).

A solution 500 ml of the compound 0.5 mol in THF was carefully added dropwise to a mixture of THF 50 ml and magnesium (0.55 mol) to prepare a solution of a Grignard reagent. A solution 300 ml of cyclohexanedion monoethyleneketal (0.55 mol) in THF was added dropwise at a temperature of 10 °C or less to the solution. The temperature of the solution was returned to room temperature and the solution was stirred for three hours. THF 700 ml was removed at a temperature of 30 °C or less under reduced pressure. A water solution 100 ml of saturated ammonium chloride and then ethyl acetate 500 ml were added to the resulting residue. The mixture was well-stirred and separated organic phase was concentrated under reduced pressure. To this concentrated organic phase, toluene 300 ml and Amberlyst 50 g, which is an acidic ion exchange resin, were added. The mixture was refluxed five hours while the resulting water was removed, and cooled to room temperature. The reactant was thoroughly washed with water and dried over anhydrous magnesium sulfate, and the solvent was removed under reduced pressure to obtain the residue of 4-((4-butylcyclohexyl)methyloxy)phenyl-1-cyclohexen-3-one=ethyleneketal (0.36 mol), yield 72%).

To the compound 0.35 mol, toluene 100 ml, ethanol 500 ml and 5% palladium-carbon 50 g was added, and the mixture was stirred for five hours in an atmosphere of hydrogen of 4 kg/cm², and the catalyst was filtered off. The filtrate was concentrated under reduced pressure, and the resulting residue was recrystallized from ethanol to obtain 4-((4-butylcyclohexyl)methyloxy)phenyl-1-cyclohexanone=ethyleneketal (0.32 mol, yield 92%).

A mixture of the compound 0.3 mol, formic acid 50 ml and toluene 500 ml was refluxed for two hours and cooled. The toluene phase was thoroughly washed with water, dried over anhydrous magnesium sulfate, and the solvent was removed under reduced pressure. The resulting residue was purified by column chromatography (elute: toluene) to obtain 4-((4-butylcyclohexyl)methyloxy)phenyl-1-cyclohexanone (0.26 mol, yield 86%).

A solution 100 ml of the compound 0.2 mol in THF was added to the following solution; a red homogeneous solution which is obtained by adding potassium-t-butoxide (0.22 mol) to a suspension of methoxymethyltriphenylphosphonium chloride (0.22 mol) in THF 200 ml, and by stirring at a temperature of 10 °C or less for two hours; and the mixture was stirred for five hours at room temperature. After removing the solvent under reduced pressure, toluene 300 ml and water 500 ml were added, and the mixture was thoroughly stirred. Separated organic phase was concentrated under reduced pressure, heptane 500 ml was added, and the mixture was thoroughly stirred. Insolubles were filtered off and resulting filtrate was concentrated and purified by column chromatography (elute: heptane) to obtain an enolether 0.15 mol.

After adding THF 300 ml and 6M hydrochloric acid 300 ml to this enolether (0.15 mol), the mixture was stirred a whole day and night, toluene 300 ml was further added, and the mixture was thoroughly stirred. Separated organic phase was washed with water and dried over anhydrous magnesium sulfate. After removing the solvent under reduced pressure, the resulting residue was purified by column chromatography (elute: toluene) to obtain 4-((4-butylcyclohexyl)methyloxy)phenyl-1-cyclohexancarbaldehyde (0.09 mol, yield 42% from cyclohexanone).

A solution 50 ml of the compound 0.05 mol in THF was added dropwise to the following solution, which is obtained by adding potassium-t-butoxide(0.055 mol) to a mixture of methyltriphenylphosphonium bromide (0.055 mol) and THF 100 ml and stirring them for one hour at room temperature, and the mixture was stirred for three hours. Further, after adding water 100 ml and heptane 100 ml, the mixture was thoroughly stirred. Precipitated insolubles were filtered off and the separated organic phase was dried over anhydrous magnesium sulfate. The solvent was removed under reduced pressure, the resulting residue was purified by column chromatography (elute: heptane) and by recrystallization (solvent : ethanol) to obtain the title compound of white solids (0.03 mol, yield 60%). The structure was supported well by each spectral data.
¹H-NMR: δ (ppm): 7.00 (4H, dd), 5.64-6.02 (1H, m), 4.99 (1H, t), 3.72 (2H, d), 2.41 (1H, t), 1.94-0.82 (28H, m).
GC-MS: 354 (M⁺)

The compound shows a property of liquid crystals, and the phase transition points were as follows. SB-N point: 78.1 °C, and N-I point: 129.0 °C.

The following compounds can be prepared according to the above method of Example 3 (No.22 - No.140).

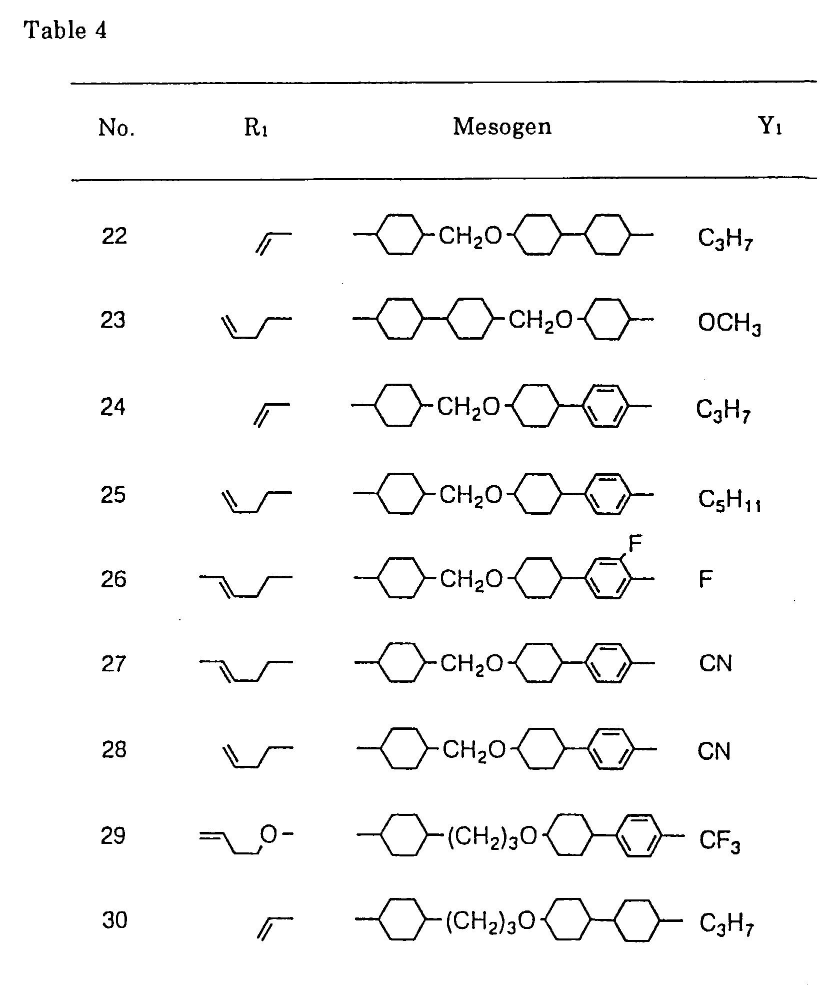

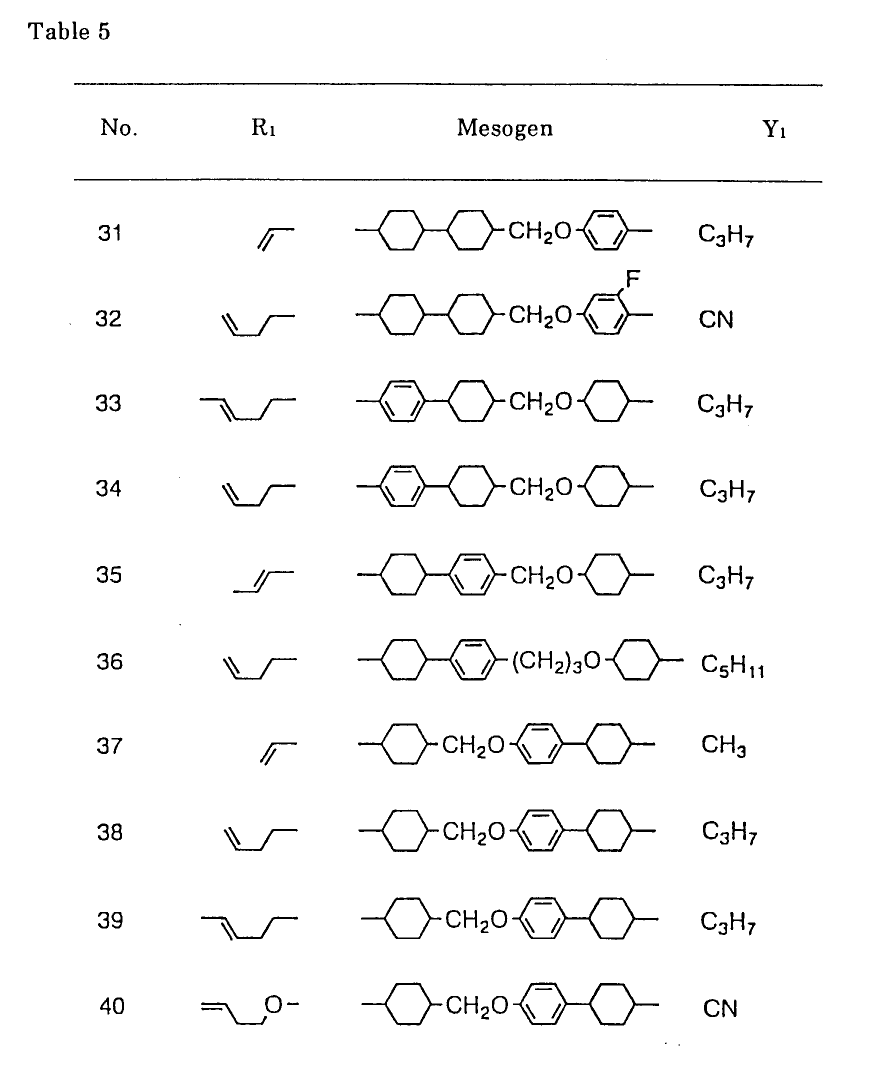

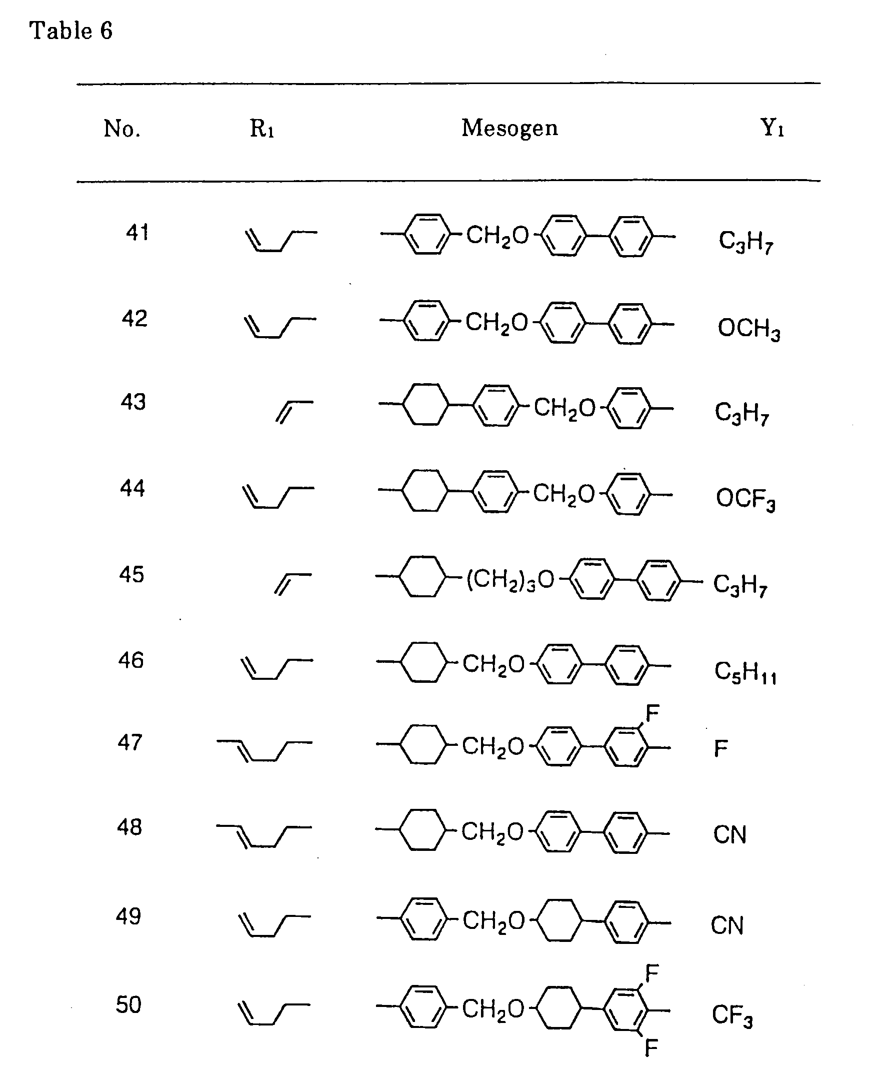

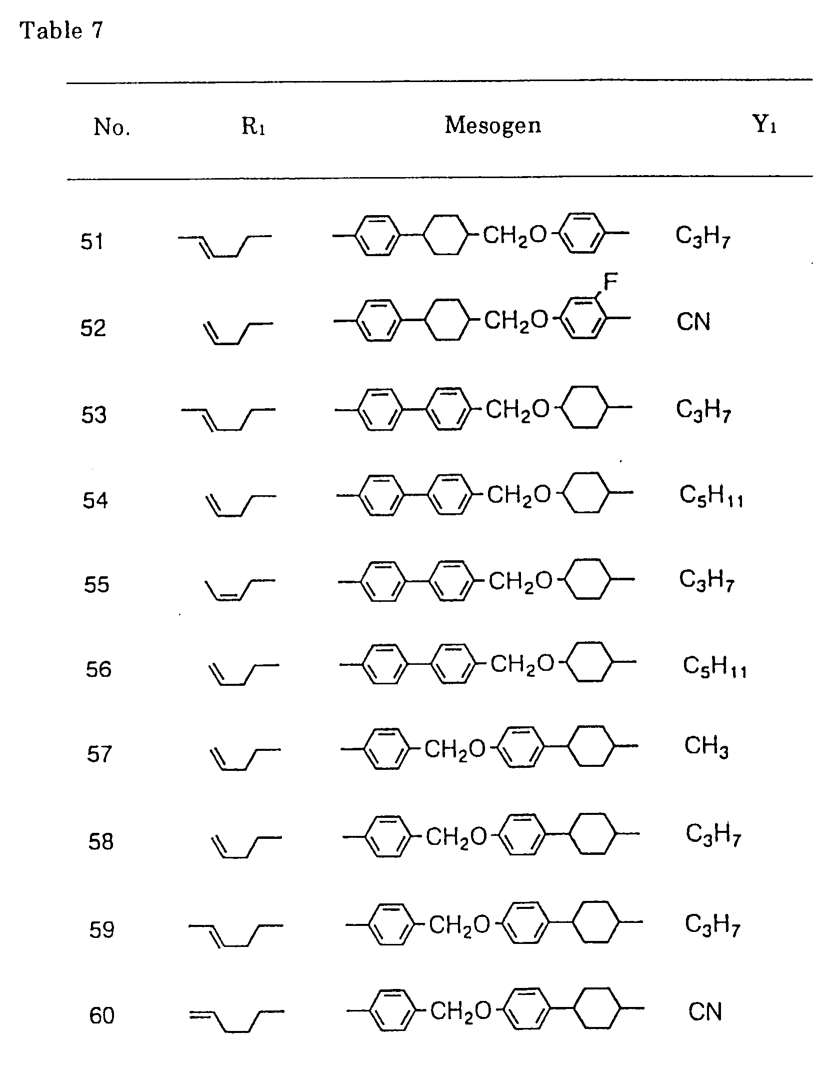

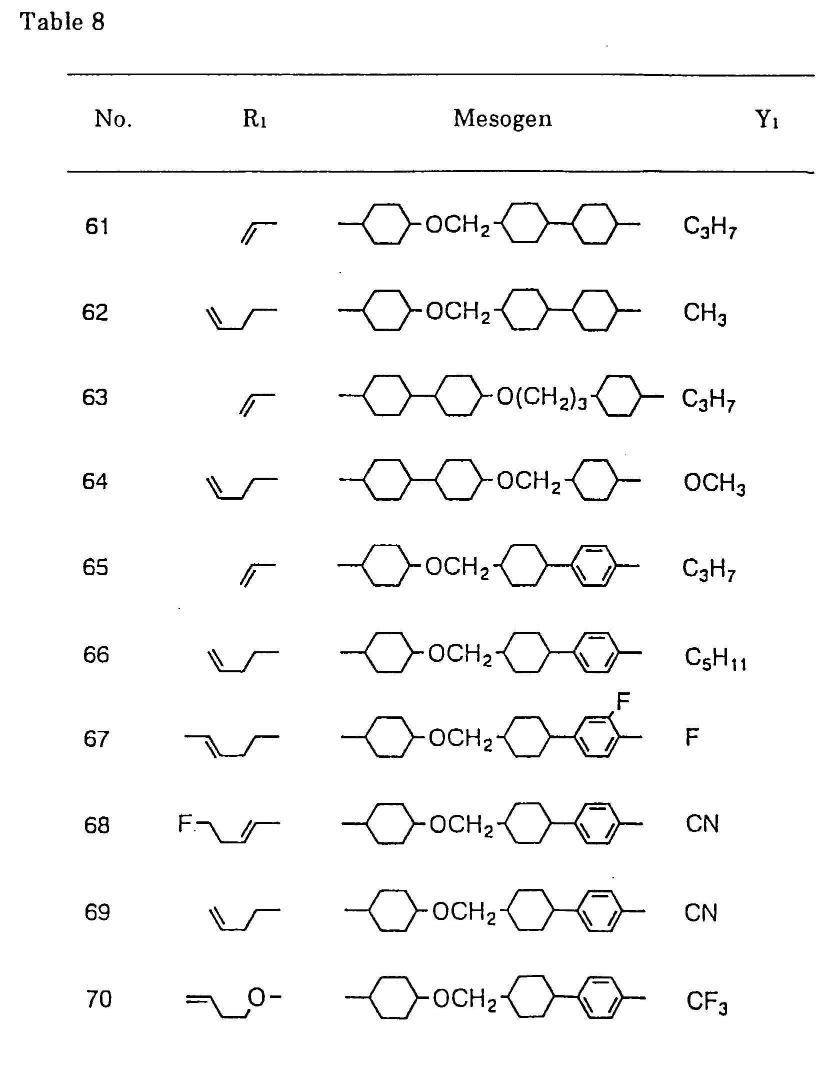

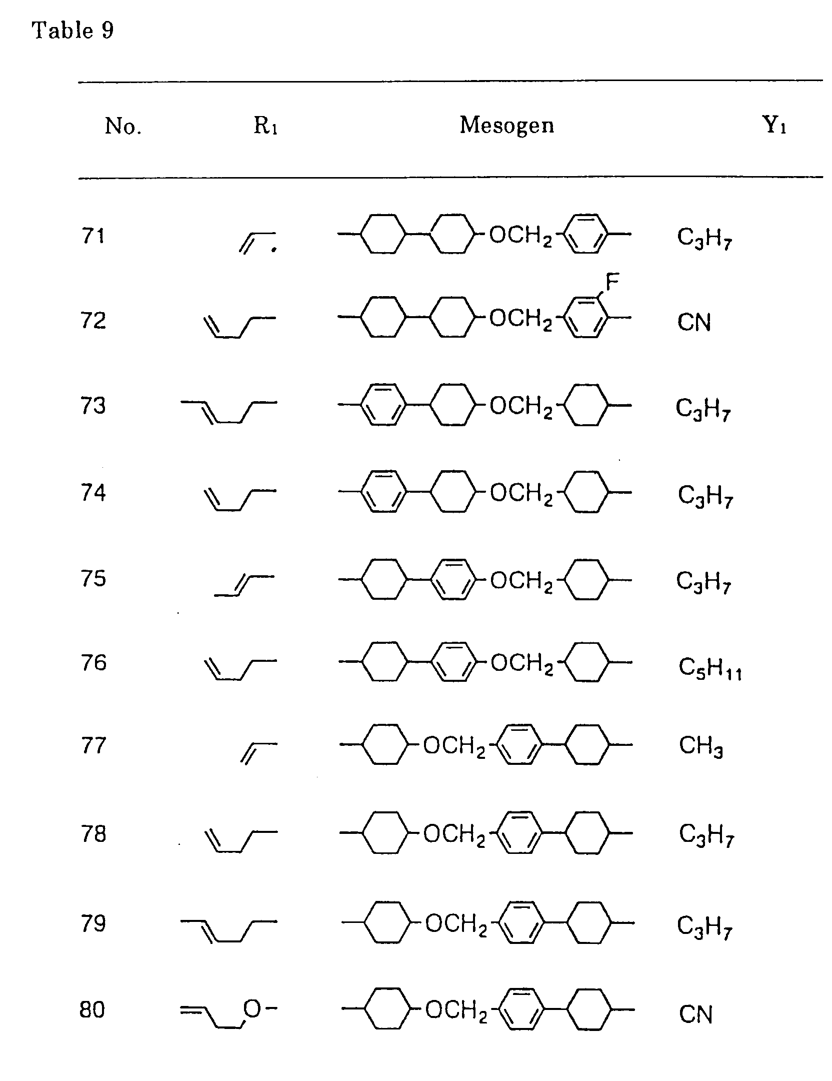

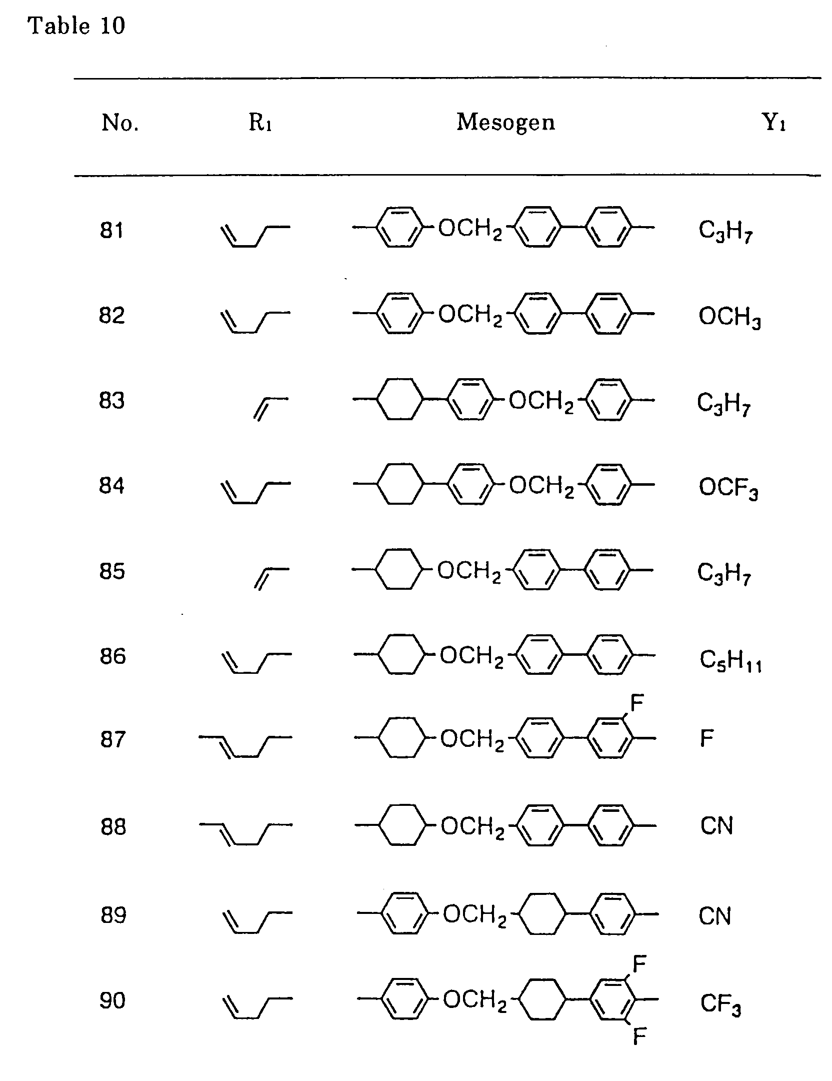

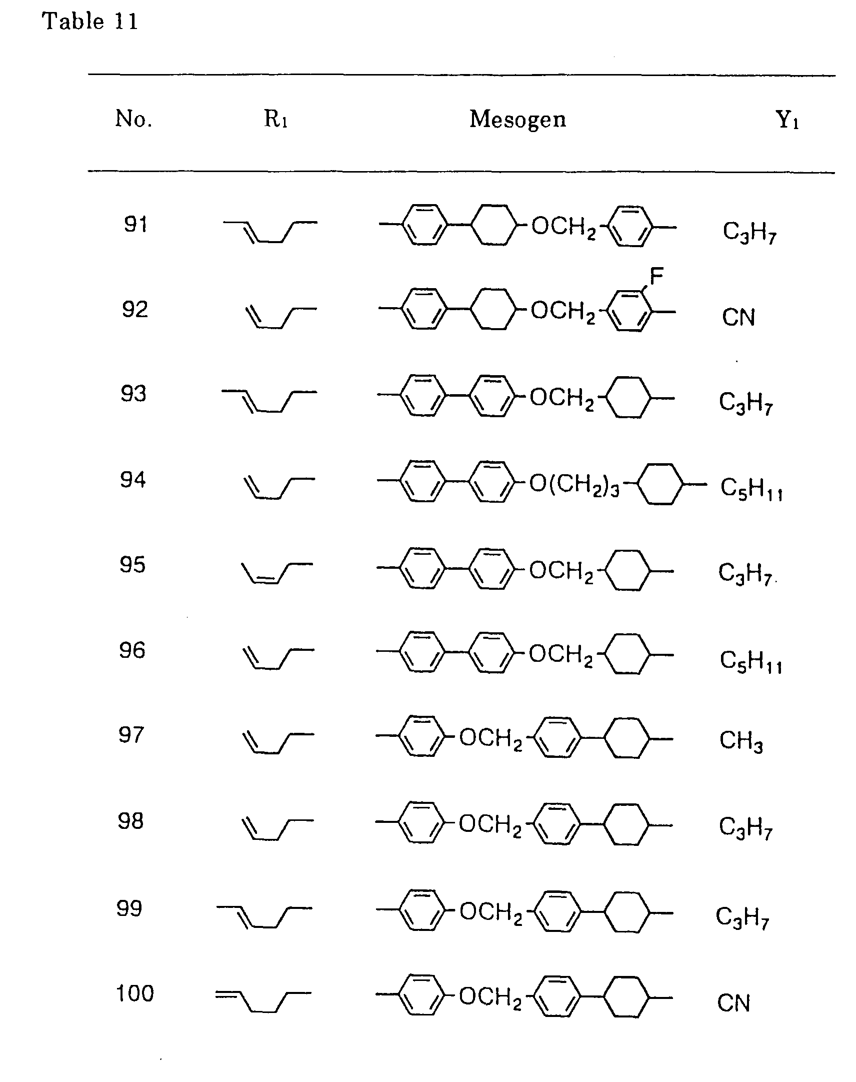

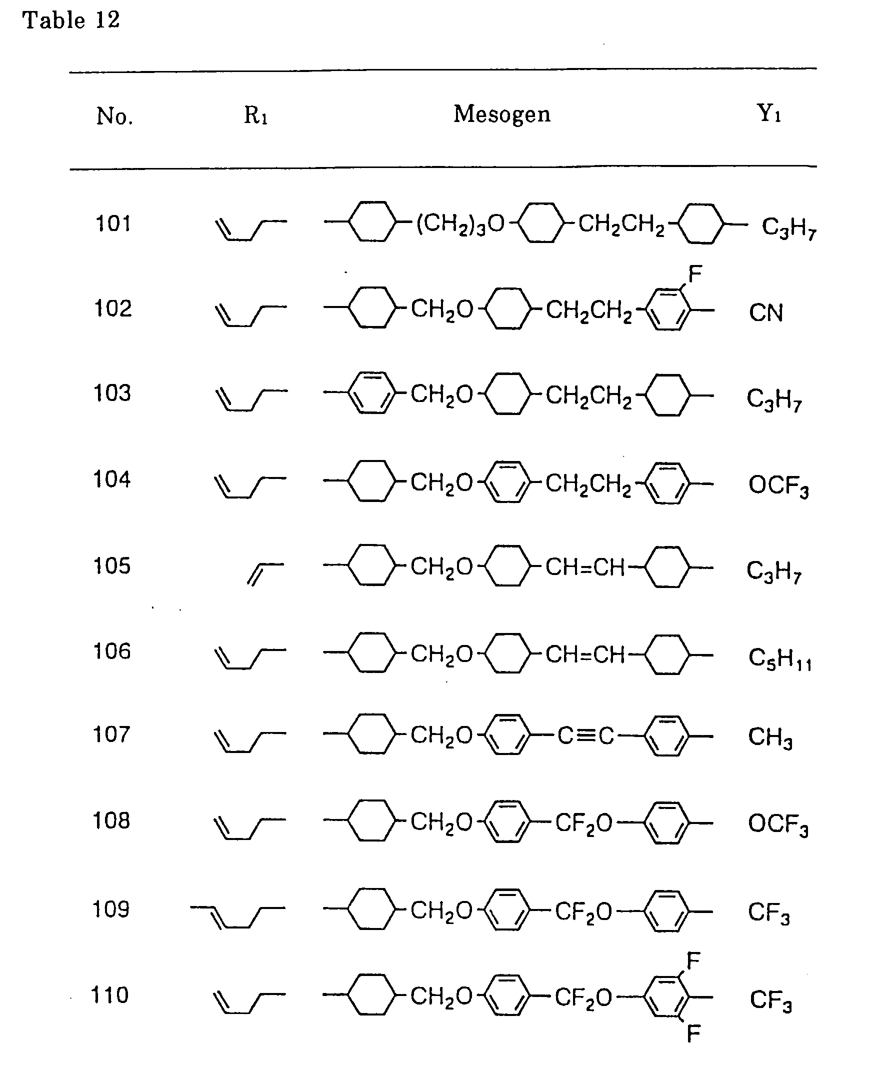

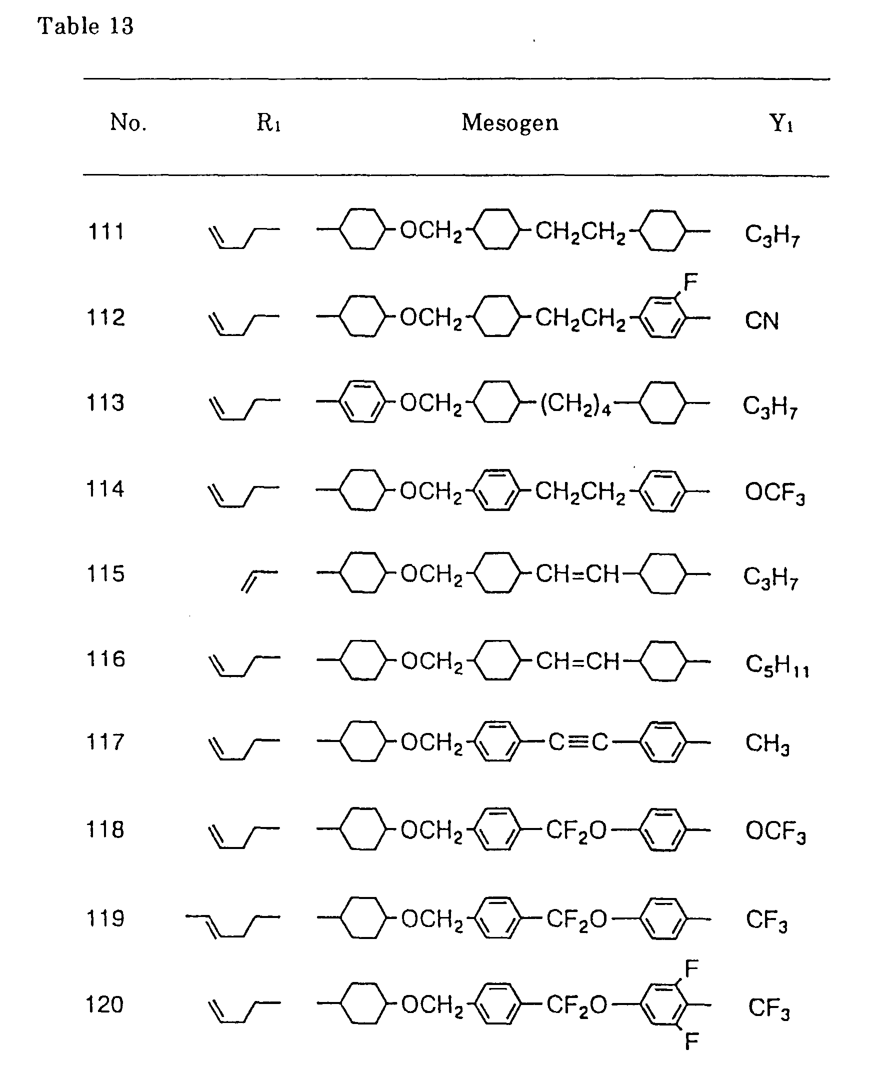

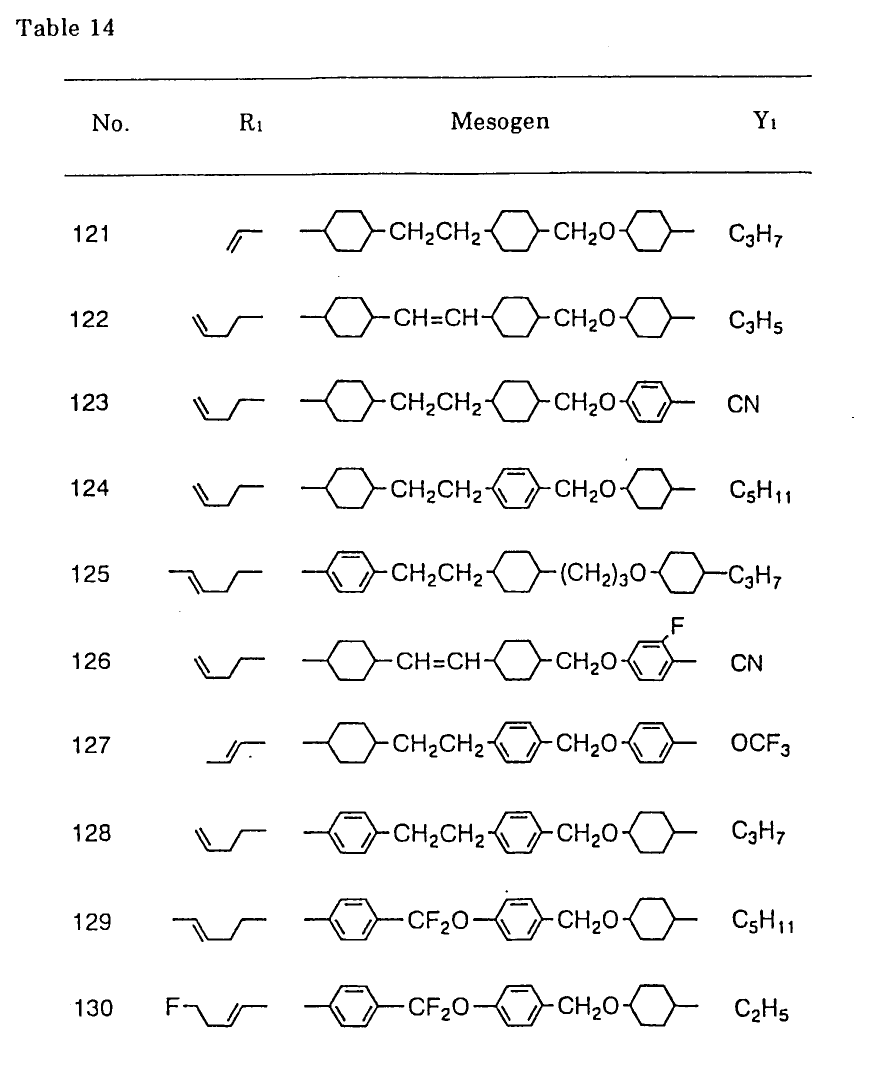

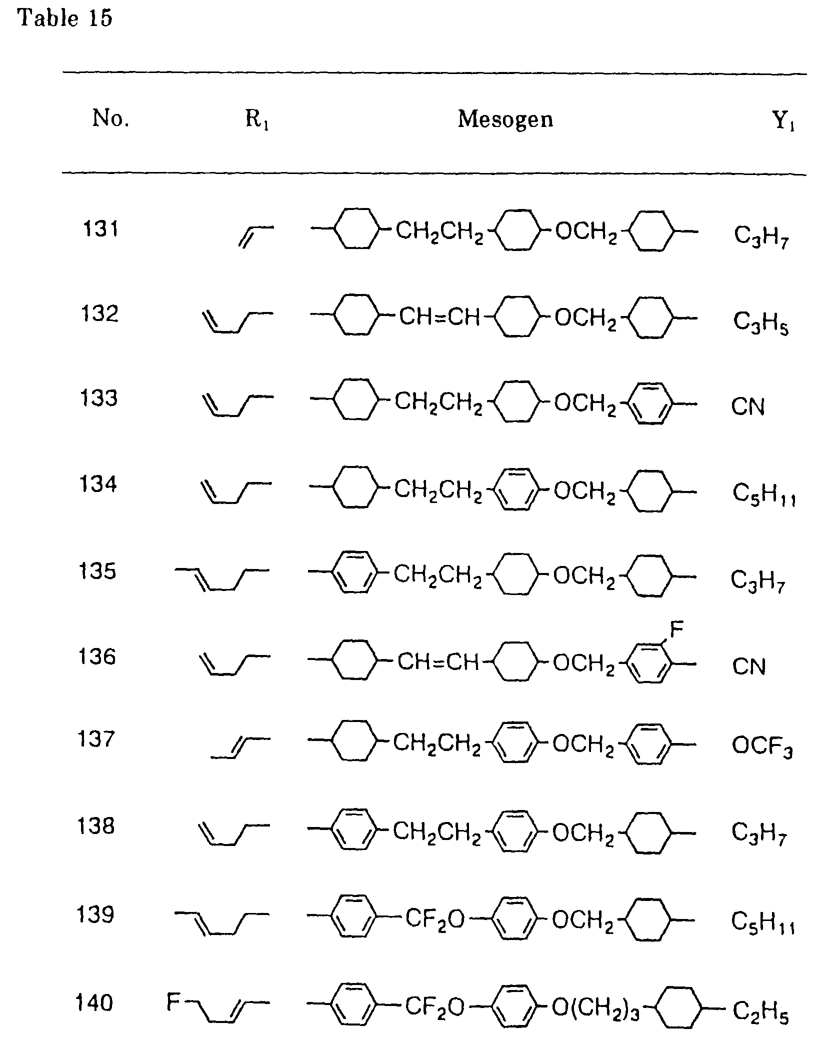

### Example 4

### Preparation of 4-(4-(4'-ethylbiphenyl-4-yl)cyclohexylmethyloxy)-1-(3-butenyl)cyclohexane (in formula (1), R₁ is a 3-butenyl group, Y₁ is an ethyl group, both ring A1 and ring A2 are 1,4-cyclohexylene, both ring A3 and A4 are 1,4-phenylene, X₁ is a oxymethylene group, both X₂, and X₃ are single bonds, and both m and n are 1 (No.141))

To a mixture, which is obtained by adding THF 200 ml and DMSO 50 ml to 4-hydroxy-7, 10-dioxy-spiro[5, 4]decane (0.4 mol) which is obtained in the former step of Example 1, sodium hydride washed well with heptane (about 0.44 mol) was added and the mixture was stirred for two hours. A THF solution 200 ml of 4-(4'-ethylbiphenyl-4-yl)cyclohexylmethylbromide (0.44 mol) was added, the mixture was stirred at 65 °C for three hours, water 300 ml and toluene 300 ml were further added, and the mixture was well-stirred. Separated organic phase was dried over anhydrous magnesium sulfate, and the solvent was removed under reduced pressure. The resulting residue was recrystallized from a mixture solvent of ethanol/ethyl acetate to obtain 4-(4-(4'-ethylbiphenyl-4-yl)cyclohexylmethyloxy)-1-cyclohexanone=ethyleneketal (0.21 mol, yield 53%). A mixture, which is obtained by adding toluene 300 ml and formic acid 50 ml to the reaction compound 0.2 mol, was refluxed for three hours and cooled to room temperature.

The reaction compound was thoroughly washed with water, and dried over anhydrous magnesium sulfate. The solvent was removed under reduced pressure, the resulting residue was recrystallized from heptane to obtain 4-(4-(4'-ethylbiphenyl-4-yl)cyclohexylmethyloxy)-1-cyclohexanone (0.18 mol, yield 90%).

A THF solution 50 ml of the compound 0.1 mol was added dropwise at a temperature of 10 °C or less to a yellow solution, which is obtained by stirring a mixture comprising of 1,3-dioxane-2-yl ethyl)triphenylphosphonium bromide (0.11 mol), potassium-t-butoxide (0.11 mol) and THF 100 ml for two hours at room temperature. The temperature was increased to room temperature and the mixture was stirred for three hours. Then, toluene 300 ml and water 300 ml were added to the reactant and well-stirred. Separated organic phase was dried over anhydrous magnesium sulfate and the solvent was removed under reduced pressure. The residue was purified by column chromatography (elute: heptane) to obtain a Wittig adduct (0.07 mol, yield 69%).

After adding toluene 200 ml and 5% palladium carbon 50 g to the compound, the mixture was stirred for five hours in a hydrogen atmosphere of 4 kg/cm². After confirming that hydrogen was not longer adsorbed, the catalyst was removed by filtration, the resulting filtrate was concentrated under reduced pressure. The residue was purified by column chromatography (elute: toluene) to obtain 4-(4-(4'-ethylbiphenyl-4-yl)cyclohexylmethyloxy)-1-(2-(1,3-dioxane-2-yl)ethyl)cyclohexane (0.022 mol, yield 32%).

A mixture of the compound 0.02 mol, toluene 50 ml and formic acid 20 ml were refluxed for two hours, cooled, and thoroughly washed with water. After drying over anhydrous magnesium sulfate, the solvent was removed under reduced pressure, and the resulting residue was recrystallized from toluene to obtain 4-(4-(4'-ethylbiphenyl-4-yl)cyclohexylmethyloxy)-1-(4-oxobutyl)cyclohexane (0.018 mol, yield 89%).

A THF solution 40 ml of the compound 0.01 mol was added dropwise to a solution, which is obtained by stirring a mixture comprising of methyltriphenyl phosphonium bromide (0.011 mol), THF 40 ml and potassium-t-butoxide (0.011 mol) for one hour at room temperature, and the mixture was stirred for three hours. Heptane 500 ml was added to the reactant to remove insolubles by filtration. The resulting filtrate was concentrated under reduced pressure. The residue was purified by column chromatography (elute: heptane) and then by recrystallization (solvent: ethanol) to obtain white solids of the title compound (0.077 mol, yield 77%). The structure was supported well by each spectral data.
GC-MS: 430 (M⁺)

The following compounds can be prepared according to the above method of Example 4 (No.142 - No.180).

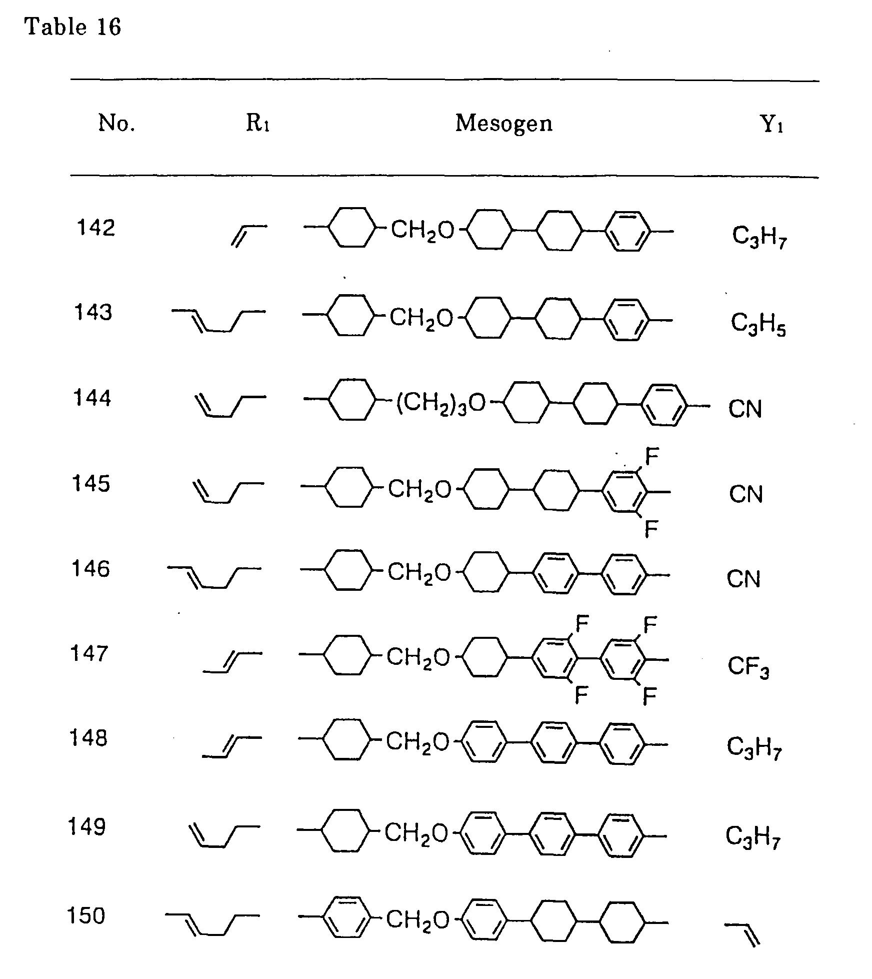

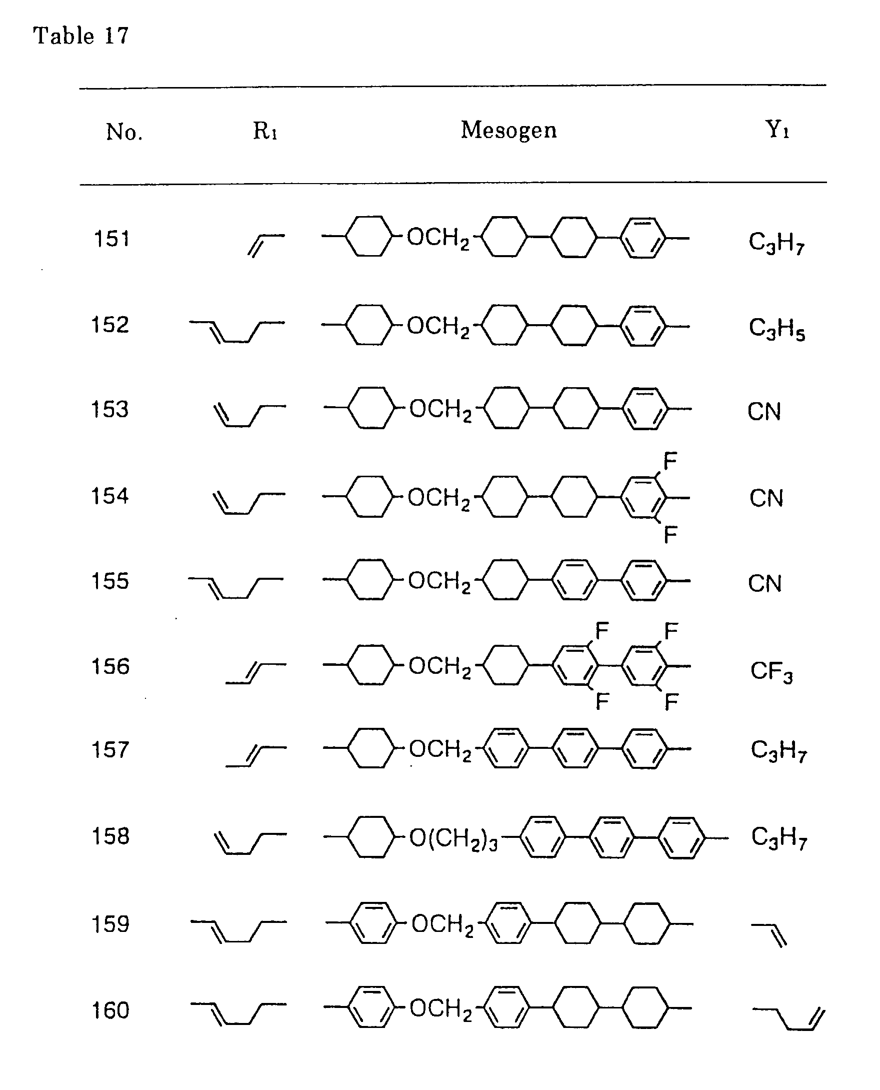

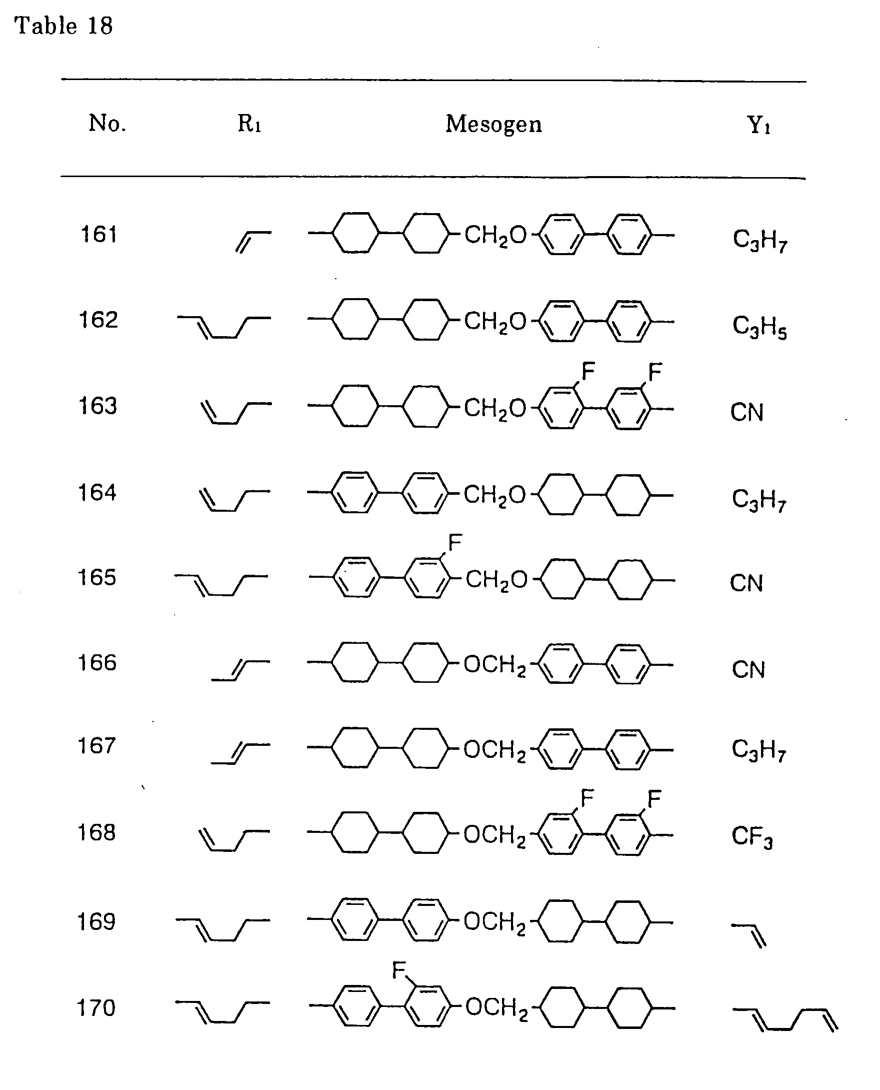

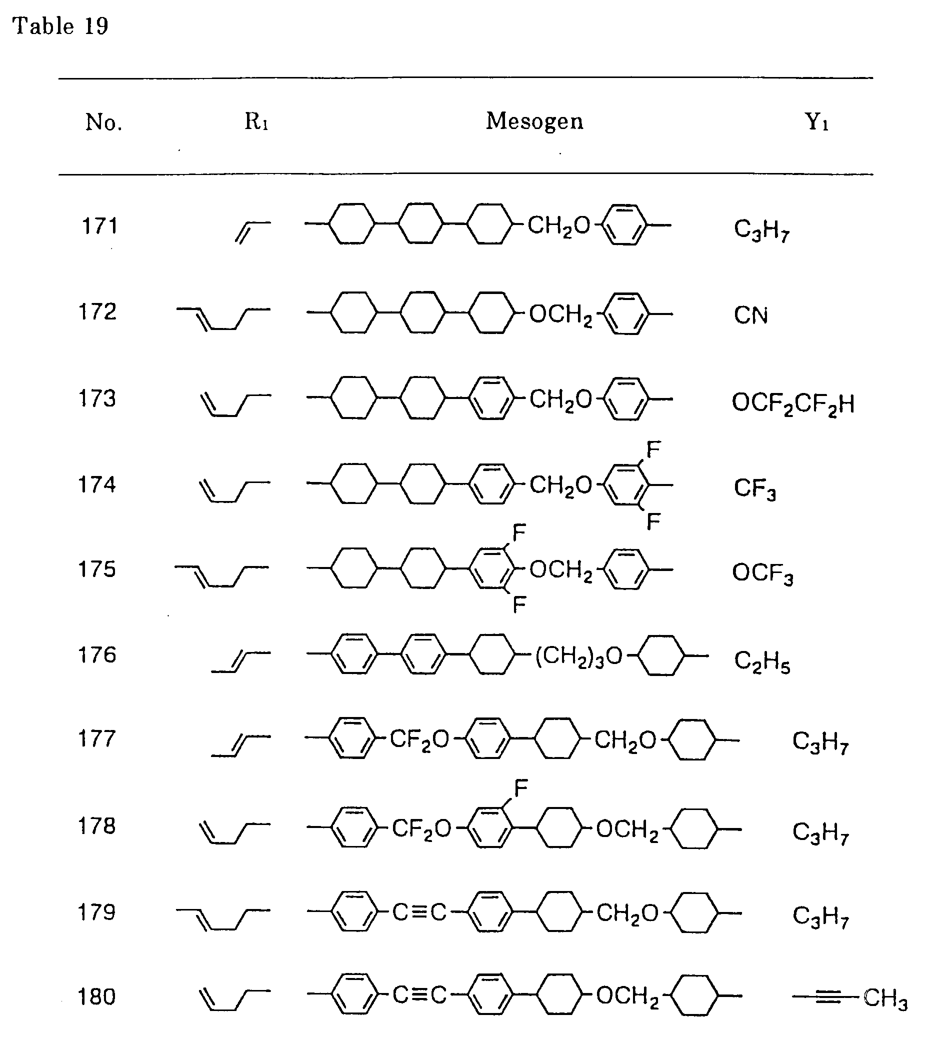

### Example 5

Liquid crystal composition A comprising of the following:

| | |
|---|---|
| 4-(4-propylcyclohexyl)benzonitrile | 24% by weight, |
| 4-(4-pentylcyclohexyl)benzonitrile | 36% by weight, |
| 4-(4-heptylcyclohexyl)benzonitrile | 25% by weight, |
| and 4-(4-propylphenyl)benzonitrile | 15% by weight, |

was prepared. The clearing point of the composition was 71.7 °C, Δε was 11.0, Δn was 0.137, the viscosity was 27 mPa · s at 20 °C, and Vₜₕ was 1.78 V at a cell thickness of 8.8 µm.

The compound of the present invention obtained in Example 3, 4-((4-butylcyclohexyl)methyloxy)phenyl-1-vinylcyclohexane (No.21) (15% by weight) was mixed with this liquid crystal composition A (85% by weight) to prepare liquid crystal composition B. The clearing point of the composition B was 79.7 °C (extrapolation value: 121.1 °C), Δε was 9.6 (extrapolation value: 1.7), Δn was 0.129 (extrapolation value: 0.084), the viscosity was 29.2 mPa · s at 20 °C (extrapolation value: 41.7 mPa · s), Vₜₕ was 1.79 V at a cell thickness of 8.8 µm, and the elastic constant ratio (K₃₃/K₁₁) was 2.67. Moreover, although the composition B was allowed to stand in a freezer at -20 °C for 60 days, the precipitation of crystals was not found.

### Comparison example 1

Liquid crystal composition C was prepared by the same method as in Example 5 except that the compound to be mixed with the composition A was changed to the compound represented by aforementioned formula (10) from the compound of No. 21 . The clearing point of the resulting composition C was 76.0 °C (extrapolation value: 100.3 °C), Δε was 13.7 (extrapolation value: 29.0), Δn was 0.147 (extrapolation value: 0.204), the viscosity was 34.0 mPa · s at 20 °C (extrapolation value: 74 mPa · s), and K₃₃/K₁₁ was 2.42. Moreover, the composition C was allowed to stand in a freezer at -20 °C, and the precipitation of crystals was found within 15 days.

### Industry Applicability

As described above, the compounds of the present invention have characteristics, such as a steep Vₜₕ property because of a large value of K₃₃/K₁₁, good miscibility to the other liquid crystalline compound, low viscosity, rapid responsibility, and appropriate magnitude of Δn and Δε, and these are suitable to liquid crystalline compounds for STN.

Accordingly, when the compounds of the present invention are used as components of liquid crystal compositions, since such compounds have excellent compatibility with the other liquid crystal materials, it is possible to provide new liquid crystal compositions having desired physical properties by selecting six membered rings, substituent groups and/or bonding groups of molecular-constituting elements.

## Claims

1. An alkenyl compound represented by the general formula (1): 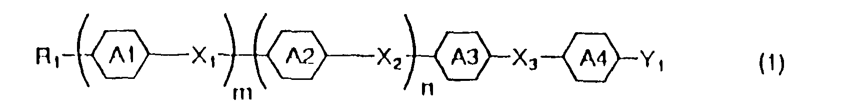 wherein
X₁, X₂, and X₃ each independently represents -CH₂O-, -OCH₂-, a single bond, -CF₂O-, -OCF₂-, -CH=CH-, -CH₂CH₂-, and at least one of them indicates -CH₂O-, or -OCH₂-;
R₁ represents an alkenyl group of 2-20 carbon atoms;
Y₁ represents an alkyl group of 1-20 carbon atoms whose methylene groups may be replaced by oxygen atoms, sulfur atoms, dihydrosilylene, dimethylsiylene or -CH≡CH-;
rings A1, A2, A3 and A4 each independently represents 1,4-cyclohexylene, or 1,4-phenylene, and carbon atoms in the rings may be replaced by oxygen atoms, and hydrogen atoms may be substituted by fluorine atoms or chlorine atoms, respectively;
m and n each independently represents 0 or 1;
provided that when at least one of X₁, X₂ and X₃ indicates -CH₂0- or -OCH₂-, then at least one of bonded rings adjacent to -CH₂0- or -OCH₂- is 1,4-cyclohexylene.

2. An alkenyl compound according to claim 1, wherein m is 0 and n is 1.

3. An alkenyl compound according to claim 1, wherein both m and n are 0.

4. A liquid crystal composition, characterized in that it comprises at least one of alkenyl compounds described in any one of claims 1 to 3.

5. A liquid crystal composition, characterized in that it comprises as the first component at least one compound selected from the group consisting of the alkenyl compounds described in any one of claims 1 to 3, and as the second component at least one compound selected from the group consisting of the compounds represented by general formulas (2), (3) and (4): 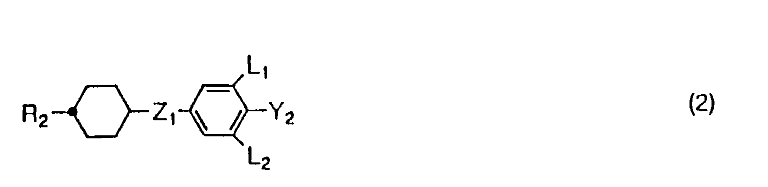 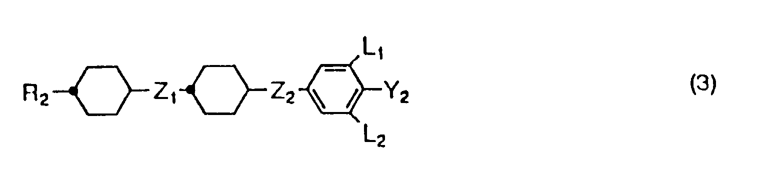 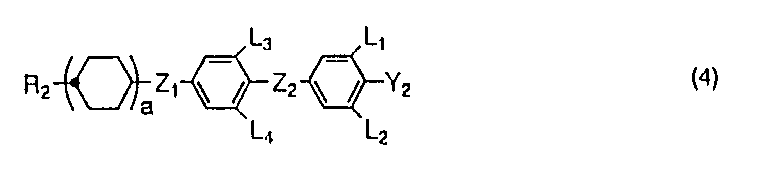 wherein R₂ represents an alkyl group having 1 to 10 carbon atoms; Y₂ represents a fluorine atom, a chlorine atom, OCF₃, OCF₂H, CF₃, CF₂H or CFH₂; L₁, L₂, L₃ and L₄ each independently represents a hydrogen atom or a fluorine atom, Z₁ and Z₂ each independently represents 1,2-ethylene group, -CH=CH- or a covalent bond, and a is 1 or 2.

6. A liquid crystal composition, characterized in that it comprises as the first component at least one compound selected from the group consisting of the alkenyl compounds described in any one of claims 1 to 3, and as the second component at least one compound selected from the group consisting of the compounds represented by general formulas (5), (G), (7), (8) and (9): 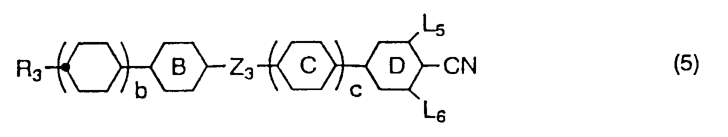 wherein R₃ represents a fluorine atom, an alkyl group having 1 to 10 carbon atoms or an alkenyl group having 2 to 10 carbon atoms, any methylene group (-CH₂-) in the alkyl group or the alkenyl group may be replaced by an oxygen atom, but two or more methylene groups are not continuously replaced by the oxygen atom; ring B represents 1,4-cyclohexylene, 1,4-phenylene or 1,3-dioxane-2, 5-diyl; ring C represents a 1,4-cyclohexylene, 1,4-phenylene or pyrimidine-2,5-diyl; ring D represents 1,4-cyclohexylene or 1,4-phenylene; Z₃ represents a 1,2-ethylene group, -COO- or a single bond; L₅ and L₆ each independently represents a hydrogen atom or a fluorine atom; and b and c each independently represents 0 or 1, 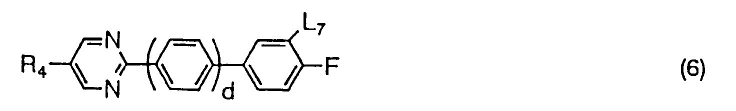 wherein R₄ represents an alkyl group having 1 to 10 carbon atoms, L₇ represents a hydrogen atom or a fluorine atom, and d is 0 or 1, 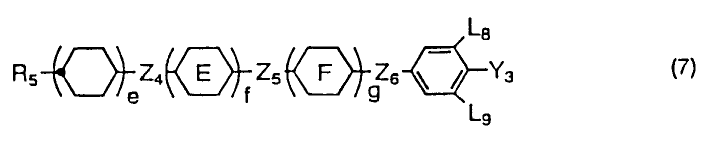 wherein R₅ represents an alkyl group having 1 to 10 carbon atoms; ring E and ring F each independently represents 1,4-cyclohexylene or 1,4-phenylene; Z₄ and Z₅ each independently represents -COO- or a single bond; Z₆ represents -COO- or -C≡C-; L₈ and L₉ each independently represents a hydrogen atom or a fluorine atom; Y₃ represents a fluorine atom, OCF₃, OCF₂H, CF₃, CF₂H or CFH₂; e, f and g each independently represents 0 or 1, 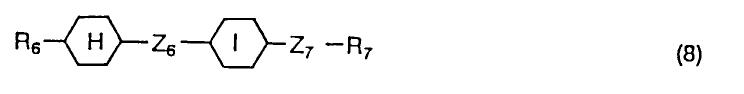 wherein R₆ and R₇ each independently represents an alkyl group having 1 to 10 carbon atoms or an alkenyl group having 2 to 10 carbon atoms, and in any case, any methylene group may be replaced by an oxygen atom, but two or more methylene groups are not continuously replaced by an oxygen atom; ring H represents 1,4-cyclohexylene, 1,4-phenylene or pyrimidine-2,5-diyl; ring I represents 1,4-cyclohexylene or 1,4-phenylene; Z₆ represents -C≡C-,-COO-, -(CH₂)₂-, -CH=CH-C≡C- or a single bond; Z₇ represents -COO- or a single bond, and 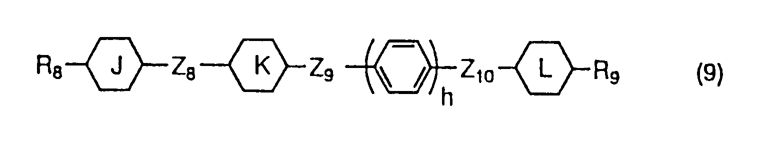 wherein R₈ and R₉ each independently represents an alkyl group having 1 to 10 carbon atoms or an alkenyl group having 2 to 10 carbon atoms, and in any case, any methylene group may be replaced by an oxygen atom, but two or more methylene groups are not continuously replaced by an oxygen atom; ring J represents 1,4-cyclohexylene, 1,4-phenylene or pyrimidine-2,5-diyl; ring K represents 1,4-cyclohexylene, 1,4-phenylene in which one or more hydrogen atoms on the ring may be substituted by a fluorine atom, or pyrimidine-2,5-diyl; ring L represents 1,4-cyclohexylene or 1,4-phenylene, Z₈ and Z₁₀ each independently represents -COO-, a 1,2-ethylene group or a single bond, Z₉ represents -CH=CH-, -C≡C-, -COO- or a covalent bond; and h represents 0 or 1.

7. A liquid crystal composition, characterized in that it comprises as the first component at least one compound selected from the group consisting of the alkenyl compounds described in any one of claims 1 to 3, and as a part of the second component at least one compound selected from the group consisting of the compounds represented by general formulas (2), (3) and (4) described in claim 5, and the other part of the second component at least one compound selected from the group consisting of the compounds represented by general formulas (5), (G), (7), (8) and (9) described in claim 6.

8. A liquid crystal display device constituted by using the liquid crystal composition described in claim 4.

9. A liquid crystal display device constituted by using the liquid crystal composition described in claim 5.

10. A liquid crystal display device constituted by using the liquid crystal composition described in claim 6.

11. A liquid crystal display device constituted by using the liquid crystal composition described in claim 7.

## Patentansprüche

1. Alkenylverbindung, representiert durch die allgemeine Formel (1): 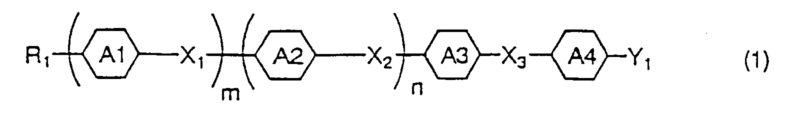 wherein
X₁, X₂ und X₃ jeweils unabhängig darstellen -CH₂0-, -OCH₂, eine Einfachbindung, -CF₂0-, -OCF₂, -CH=CH-, -CH₂CH₂- und wenigstens eines von ihnen die Bedeutung -CH₂0- oder -OCH₂- hat; R₁ ist eine Alkenylgruppe mit 2-20 Kohlenstoffatomen; Y₁ ist eine Alkylgruppe mit 1-20 Kohlenstoffatomen, deren Methylengruppen ersetzt sein können durch Sauerstoffatom, Schwefelatom, Dihydrosilylen, Dimethylsilylen oder -CH≡CH-;
die Ringe A1, A2, A3 und A4 stellen jeweils unabhängig 1,4-Cyclohexylen oder 1,4-Phenylen dar, und die Kohlenstoffatome in den Ringen können durch Sauerstoffatome ersetzt sein, und die Wasserstoffatome können durch Fluoratome oder Chloratome ersetzt sein;
m und n sind jeweils unabhängig 0 oder 1;
mit der Maßgabe, daß wenn wenigstens einer der Reste X₁, X₂ und X₃ die Bedeutung -CH₂0- oder -OCH₂- hat, wenigstens einer der in Nachbarschaft zu -CH₂0- oder -OCH₂- gebundenen Ringe ein 1,4-Cyclohexylenring ist.

2. Alkenylverbindung nach Anspruch 1, worin m die Bedeutung 0 hat, and n ist 1.

3. Alkenylverbindung nach Anspruch 1, worin sowohl m als auch n die Bedeutung 0 haben.

4. Flüssigkristallzusammensetzung, dadurch gekennzeichnet, daß sie wenigstens eine der in einem der Ansprüche 1 bis 3 beschriebenen Alkenylverbindungem umfaßt.

5. Flüssigkristallzusammensetzung, dadurch gekennzeichnet, daß sie als erste Komponente wenigstens eine Verbindung enthält, die aus der Gruppe ausgewählt ist, bestehend aus den in einem der Ansprüche Ansprüche 1 bis 3 beschriebenen Alkenylverbindungen, und als zweite Komponente wenigstens eine Verbindung enthält, die aus der Gruppe ausgewählt ist, bestehend aus den durch die folgenden Formeln (2), (3) und (4) repräsentierten Verbindungen: 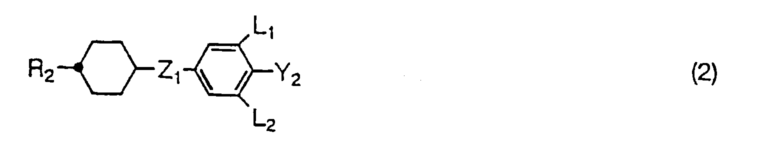 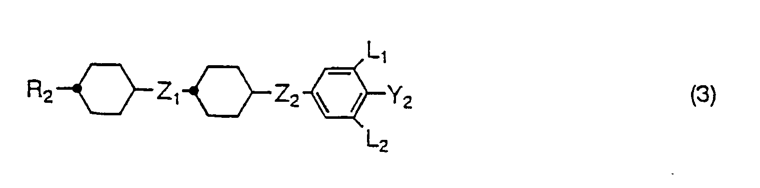 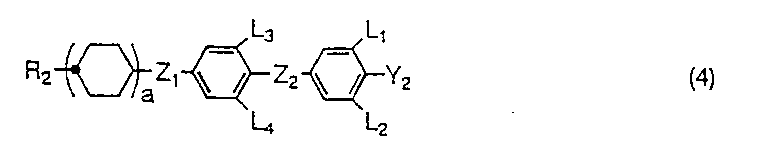 worin R₂ eine Alkylgruppe mit 1 bis 10 Kohlenstoffatomen ist; Y₂ ist ein Fluoratom, ein Chloratom, OCF₃, OCF₂H, CF₃, CF₂H oder CFH₂; L₁, L₂, L₃ und L₄ stellen jeweils unabhängig voneinander ein Wasserstoffatom oder ein Fluoratom dar, Z₁ und Z₂ stellen jeweils unabhängig voneinander eine 1,2-Ethylengruppe, -CH=CH- oder eine kovalente Bindung dar, und a ist 1 oder 2.

6. Flüssigkristallzusammensetzung, dadurch gekennzeichnet, daß sie als erste Komponente wenigstens eine Verbindung enthält, die aus der Gruppe ausgewählt ist, bestehend aus den in einem der Ansprüche Ansprüche 1 bis 3 beschriebenen Alkenylverbindungen, und als zweite Komponente wenigstens eine Verbindung enthält, die aus der Gruppe ausgewählt ist, bestehend aus den durch die folgenden Formeln (5), (6), (7), (8) und (9) repräsentierten Verbindungen: 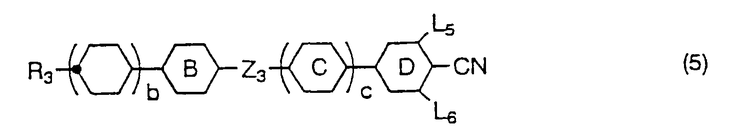 wherein R₃ ein Fluoratom, eine Alkylgruppe mit 1 bis 10 Kohlenstoffatomen oder eine Alkenylgruppe mit 2 bis 10 Kohlenstoffatomen, wobei eine beliebige Methylengruppe (-CH₂-) in der Alkylgruppe oder in der Alkenylgruppe durch ein Sauerstoffatom ersetzt sein kann, jedoch können nicht zwei oder mehr Methylengruppen zusammenhängend durch das Sauerstoffatom ersetzt werden; der Ring B ist 1,4-Cyclohexylen, 1,4-Phenylen oder 1,3-Dioxan-2,5-diyl; der Ring C ist 1,4-Cyclohexylen, 1,4-Phenylen oder Pyrimidin-2,5-diyl; der Ring D ist 1,4-Cyclohexylen oder 1,4-Phenylen; Z₃ ist eine 1,2-Ethylengruppe, -COO- oder eine Einfachbindung, L₅ und L₆ sind jeweils unabhängig voneinander ein Wasserstoffatom oder ein Fluoratom; und b und c sind jeweils unabhängig voneinander 0 oder 1, 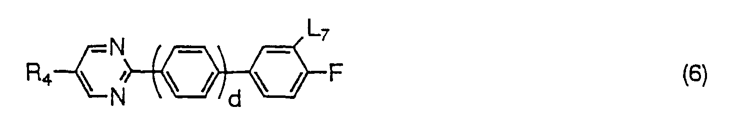 worin R₄ eine Alkylgruppe mit 1 bis 10 Kohlenstoffatomen darstellt, L₇ ist ein Wasserstoffatom oder ein Fluoratom, und d ist 0 oder 1, 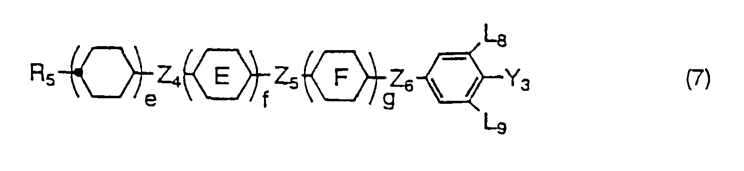 worin R₅ eine Alkylgruppe mit 1 bis 10 Kohlenstoffatom ist; der Ring E und
der Ring F sind jeweils unabhängig 1,4-Cyclohexylen oder 1,4-Phenylen;
Z₄ and Z₅ sind jeweils unabhängig -COO- oder eine Einfachbindung; Z₆ ist -COO- oder -C≡C-; und L₈ und L₉ sind jeweils unabhängig voneinander ein Wasserstoffatom oder ein Fluoratom; Y₃ ist ein Fluoratom, OCF₃, OCF₂H, CF₃, CF₂H oder CFH₂; e, f und g sind jeweils unabhängig voneinander 0 oder 1, 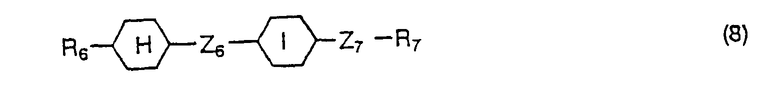 worin R₆ und R₇ jeweils unabhängig eine Alkylgruppe mit 1 bis 10 Kohlenstoffatomen oder eine Alkenylgruppe mit 2 bis 10 Kohlenstoffatomen darstellen, und in jedem Fall kann eine beliebige Methylengruppe durch ein Sauerstoffatom ersetzt sein, jedoch können nicht zwei oder mehr Methylengruppen zusammenhängend durch ein Sauerstoffatom ersetzt werden; der Ring H ist 1,4-Cyclohexylen, 1,4-Phenylen oder Pyrimidin-2,5-diyl; der Ring I ist 1,4-Cyclohexylen oder 1,4-Phenylen; Z₆ ist -C≡C-, -COO-, -(CH₂)₂-, CH=CH-C≡C- oder eine Einfachbindung; Z₇ ist -COO- oder eine Einfachbindung, und 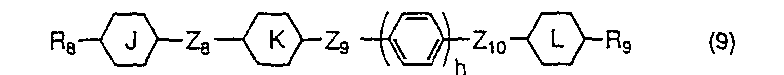 worin R₈ und R₉ jeweils unabhängig eine Alkylgruppe mit 1 bis 10 Kohlenstoffatomen oder eine Alkenylgruppe mit 2 bis 10 Kohlenstoffatomen darstellen, und in jedem Fall kann eine beliebige Methylengruppe durch ein Sauerstoffatom ersetzt werden, jedoch können nicht zwei oder mehr Methylengruppen zusammenhängend durch ein Sauerstoffatom ersetzt werden; der Ring J ist 1,4-Cyclohexylen, 1,4-Phenylen oder Pyrimidin-2,5-diyl; der Ring K ist 1,4-Cyclohexylen, 1,4-Phenylen, worin ein oder mehrere Wasserstoffatome am Ring durch ein Fluoratom substituiert sein können, oder Pyrimidin-2,5-diyl; der Ring L ist 1,4-Cyclohexylen oder 1,4-Phenylen; Z₈ und Z₁₀ sind jeweils unabhängig -COO-, eine 1,2-Ethylengruppe oder eine Einfachbindung, Z₉ ist -CH=CH-, -C≡C-, -COO- oder eine kovalente Bindung; und h ist 0 oder 1.

7. Flüssigkristallzusammensetzung, dadurch gekennzeichnet, daß sie als erste Komponente wenigstens eine Verbindung enthält, die aus der Gruppe ausgewählt ist, bestehend aus den in einem der Ansprüche 1 bis 3 beschriebenen Alkenylverbindungen,
und als einen Teil der zweiten Komponente wenigstens eine Verbindung enthält, die aus der Gruppe ausgewählt ist, bestehend aus den durch die allgemeinen Formeln (2), (3) und (4) in Anspruch 5 beschriebenen Verbindungen, und als den anderen Teil der zweiten Komponente wenigstens eine Verbindung enthält, die aus der Gruppe ausgewählt ist, bestehend aus den durch die allgemeinen Formeln (5), (6), (7), (8) und (9) in Anspruch 6 beschriebenen Verbindungen.

8. Flüssigkristall-Anzeigeelement, gebildet unter Verwendung der in Anspruch 4 beschriebenen Flüssigkristallzusammensetzung.

9. Flüssigkristall-Anzeigeelement, gebildet unter Verwendung der in Anspruch 5 beschriebenen Flüssigkristallzusammensetzung.

10. Flüssigkristall-Anzeigeelement, gebildet unter Verwendung der in Anspruch 6 beschriebenen Flüssigkristallzusammensetzung.

11. Flüssigkristall-Anzeigeelement, gebildet unter Verwendung der in Anspruch 7 beschriebenen Flüssigkristallzusammensetzung.

## Revendications

1. Composé alcényle représenté par la formule générale (1) 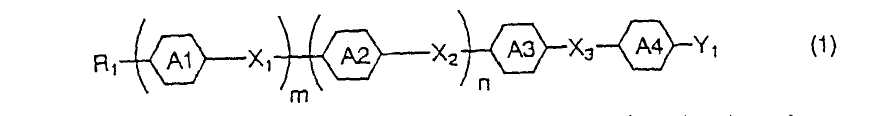 dans laquelle
X₁, X₂ et X₃ représentent chacun indépendamment -CH₂0-, -OCH₂-, une liaison simple, -CF₂O-, -OCF₂-, -CH=CH-, -CH₂CH₂-, et au moins l'un d'eux indique -CH₂0- ou -OCH₂-;
R₁ représente un groupement alcényle de 2-20 atomes de carbone;
Y₁ représente un groupement alkyle de 1-20 atomes de carbone dont les groupes méthylène peuvent être remplacés par des atomes d'oxygène, des atomes de soufre, du dihydrosilylène, du diméthylsilylène ou-CH≡CH-;
les anneaux A1, A2, A3 et A4 représentent chacun indépendamment le 1,4-cyclohexylène ou le 1,4-phénylène, et les atomes de carbone présents dans les anneaux peuvent être remplacés par des atomes d'oxygène, et les atomes d'hydrogène peuvent être remplacés par des atomes de fluor ou des atomes de chlore, respectivement;
m et n représentent, chacun indépendamment, 0 ou 1;
à la condition que si au moins l'un de X₁, X₂ ou X₃ indique -CH₂0- ou -OCH₂-, alors au moins l'un des anneaux liés adjacents à -CH₂0- ou -OCH₂- est le 1,4-cyclohexylène.

2. Composé alcényle selon la revendication 1, dans lequel m est 0 et n est 1.

3. Composé alcényle selon la revendication 1, dans lequel m et n sont tous deux 0.

4. Composition cristalline liquide, caractérisée en ce qu'elle comprend au moins l'un des composés alcényles décrit dans l'une ou l'autre des revendications 1 à 3.

5. Composition cristalline liquide, caractérisée en ce qu'elle comprend, comme premier composant, au moins un composé sélectionné dans le groupe comprenant les composés alcényles décrits dans l'une des revendications 1 à 3, et comme second composant au moins un composé sélectionné dans le groupe comprenant les composés représentés par les formules générales (2), (3) et (4) : 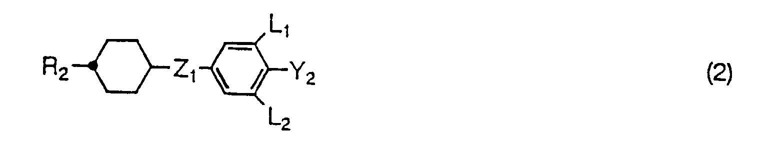 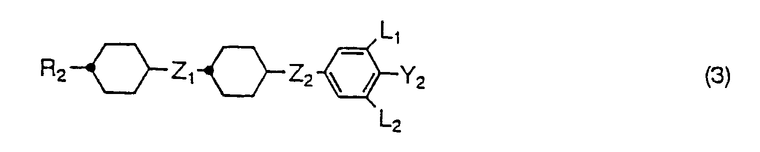 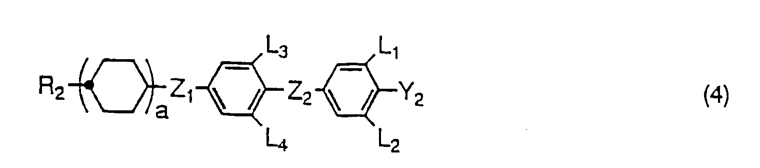 dans lesquelles R₂ représente un groupement alkyle possédant 1 à 10 atomes de carbone; Y₂ représente un atome de fluor, un atome de chlore, OCF₃, OCF₂H, CF₃, CF₂H ou CFH₂; L₁, L₂, L₃ et L₄ représentent, chacun indépendamment, un atome d'hydrogène ou un atome de fluor, Z₁ et Z₂ représentent, chacun indépendamment, un groupement 1,2-éthylène, -CH=CH- ou une liaison covalente, et a est 1 ou 2.

6. Composition cristalline liquide, caractérisée en ce qu'elle comprend, comme premier composant, au moins un composé sélectionné dans le groupe comprenant les composés alcényles décrits dans l'une des revendications 1 à 3, et comme second composant, au moins un composé sélectionné dans le groupe comprenant les composés représentés par les formules générales (5), (6), (7), (8) et (9) : 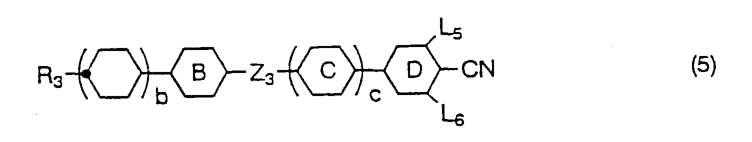 où R₃ représente un atome de fluor, un groupement alkyle possédant 1 à 10 atomes de carbone ou un groupement alcényle possédant 2 à 10 atomes de carbone, n'importe quel groupement méthylène (-CH₂-) dans le groupement alkyle ou dans le groupement alcényle peut être remplacé par un atome d'oxygène, mais deux groupements méthylène ou plus ne sont pas remplacés continuellement par l'atome d'oxygène; l'anneau B représente le 1,4-cyclohexylène, le 1,4-phénylène ou le 1,3-dioxane-2,5-diyle, l'anneau C représente un 1,4-cyclohexylène, 1,4-phénylène ou pyrimidine-2,5-diyle; l'anneau D représente le 1,4-cyclohexylène ou 1,4-phénylène; Z₃ représente un groupement 1,2-éthylène, -COO- ou une liaison simple; L₅ et L₆ représentent, chacun indépendamment, un atome d'hydrogène ou un atome de fluor; et b et c représentent chacun indépendamment 0 ou 1, 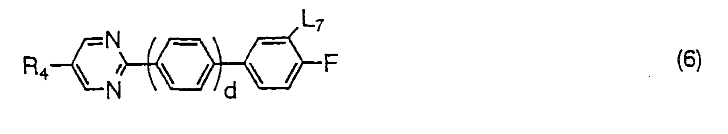 où R₄ représente un groupement alkyle possédant 1 à 10 atomes de carbone, L₇ représente un atome d'hydrogène ou un atome de fluor, et d est 0 ou 1, 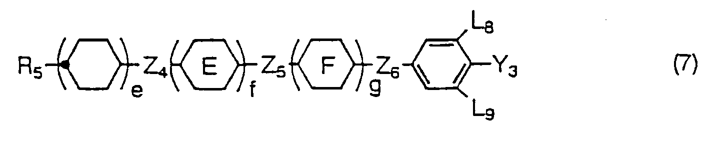 où R₅ représente un groupement alkyle possédant 1 à 10 atomes de carbone; l'anneau E et l'anneau F représentent, chacun indépendamment, le 1,4-cyclohexylène ou le 1,4-phénylène; Z₄ et Z₆ représentent, chacun indépendamment, -COO- ou une liaison simple; Z₆ représente -COO- ou -C≡C-; L₈ et L₉ représentent, chacun indépendamment, un atome d'hydrogène ou un atome de fluor; Y₃ représente un atome de fluor; OCF₃, OCF₂H, CF₃, CF₂H ou CFH₂; e, f et g représentent, chacun indépendamment, 0 ou 1, 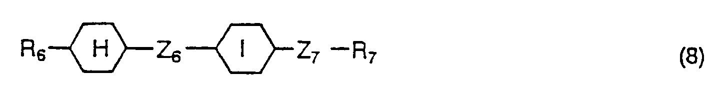 où R₆ et R₇ représentent, chacun indépendamment, un groupement alkyle possédant 1 à 10 atomes de carbone ou un groupement alcényle possédant 2 à 10 atomes de carbone, et dans tous les cas, tout groupement méthylène peut être remplacé par un atome d'oxygène, mais deux groupements méthylène ou plus ne sont pas remplacés continuellement par un atome d'oxygène; l'anneau H représente le 1,4-cyclohexylène, le 1,4-phénylène ou la pyrimidine-2,5-diyle; l'anneau I représente le 1,4-cyclohexylène ou 1,4-phénylène; Z₆ représente -C≡C-, -COO-, -(CH₂)₂-, -CH=CH-C≡C- ou une liaison simple; Z₇ représente -COO- ou une liaison simple; et 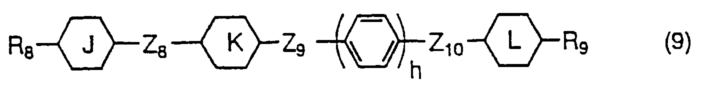 où R₈ et R₉ représentent, chacun indépendamment, un groupement alkyle possédant 1 à 10 atomes de carbone ou un groupement alcényle possédant 2 à 10 atomes de carbone, et dans tous les cas, tout groupement méthylène peut être remplacé par un atome d'oxygène, mais deux groupements méthylène ou plus ne sont pas remplacés continuellement par un atome d'oxygène; l'anneau J représente le 1,4-cyclohexylène, le 1,4-phénylène ou la pyrimidine-2,5-diyle; l'anneau K représente le 1,4-cyclohexylène, le 1,4-phénylène dans lequel un atome d'hydrogène ou plus présent sur l'anneau peut être remplacé par un atome de fluor, ou la pyrimidine-2,5-diyle; l'anneau L représente le 1,4-cyclohexylène ou le 1,4-phénylène, Z₈ et Z₁₀ représentent, chacun indépendamment, -COO-, un groupement 1,2-éthylène ou une liaison simple, Z₉ représente -CH=CH-, -C≡C-, -COO- ou une liaison covalente; et h représente O ou 1.

7. Composition cristalline liquide, caractérisée en ce qu'elle comprend, comme premier composant, au moins un composé sélectionné dans le groupe comprenant les composés alcényles décrits dans l'une des revendications 1 à 3, et comme partie du second composant, au moins un composé sélectionné dans le groupe comprenant les composés représentés par les formules générales (2), (3) et (4) décrits dans la revendication 5, et comme autre partie du second composant, au moins un composé sélectionné dans le groupe comprenant les composés représentés par les formules générales (5), (6), (7), (8) et (9) décrits dans la revendication 6.

8. Dispositif d'affichage à cristaux liquides créé par mise en oeuvre de la composition cristalline liquide décrite dans la revendication 4.

9. Dispositif d'affichage à cristaux liquides créé par mise en oeuvre de la composition cristalline liquide décrite dans la revendication 5.

10. Dispositif d'affichage à cristaux liquides créé par mise en oeuvre de la composition cristalline liquide décrite dans la revendication 6.

11. Dispositif d'affichage à cristaux liquides créé par mise en oeuvre de la composition cristalline liquide décrite dans la revendication 7.
